(19) Europäisches Patentamt / European Patent Office / Office européen des brevets

(11) EP 3 252 055 B1

(12) **EUROPEAN PATENT SPECIFICATION**

(45) Date of publication and mention of the grant of the patent: **19.09.2018 Bulletin 2018/38**

(51) Int Cl.: ***C07D 489/08*** *(2006.01)*

(21) Application number: **16382245.5**

(22) Date of filing: **31.05.2016**

(54) **PROCESS FOR OBTAINING 3,14-DIACETYLOXYMORPHONE FROM ORIPAVINE**

VERFAHREN ZUR GEWINNUNG VON 3,14-DIACETYLOXYMORPHON AUS ORIPAVIN

PROCÉDÉ D'OBTENTION DE 3,14-DIACETYLOXYMORPHONE À PARTIR D'ORIPAVINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application: **06.12.2017 Bulletin 2017/49**

(73) Proprietor: **Alcaliber Investigacion Desarrollo e Innovacion SLU**
**02007 Albacete (ES)**

(72) Inventors:
- **MITCHELL, Melville**
  **Edinburgh, EH10 6AS (GB)**
- **VÁZQUEZ CRUZ, Santiago**
  **E-08940 Cornellá (ES)**
- **LUKACH CASTORE, Andrés E.**
  **E-08015 Barcelona (ES)**
- **LOZANO VENTURA, Oscar**
  **E-08014 Barcelona (ES)**
- **CASTAÑE ABRADO, Joan**
  **E-08490 Tordera (ES)**

(74) Representative: **ABG Patentes, S.L.**
**Avenida de Burgos, 16D**
**Edificio Euromor**
**28036 Madrid (ES)**

(56) References cited:
**WO-A1-99/02529**
**WO-A1-2011/117172**
**WO-A2-2009/122436**
**US-A1- 2012 283 444**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention relates to a new process for obtaining 3,14-diacetyloxymorphone from oripavine.

### Background of the invention

The opiate alkaloids, obtained from poppy plants of the family Papaveraceae, include some of the most powerfully acting and clinically useful drugs for inducing analgesia and also are useful agents for inducing sleep in the presence of pain and as antitussive agents. Exemplary naturally-occurring opiates include morphine, codeine, thebaine and oripavine.

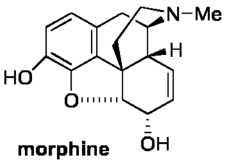
morphine

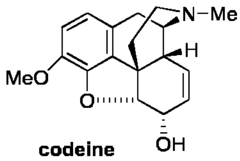
codeine

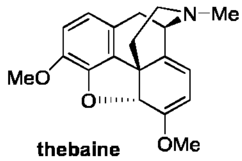
thebaine

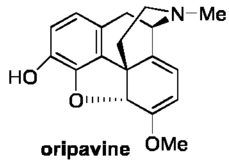
oripavine

Despite its valuable clinical properties, morphine has a large number of negative effects as it also depresses respiration, causes constipation and urinary retention. More importantly, if administered over a long period of time, the patient develops a tolerance to the analgesic effect, so the dosage must be increased, and dependence on morphine and related compounds can develop.

For these reasons, extensive efforts have been directed towards the semi-synthesis of morphine-like molecules with analgesic properties but with less undesirable addictive side effects, such as nalorphine, which retains analgesic properties but also acts as a narcotic antagonist to reverse many of the unwanted side effects of morphine. Some of them include 14-hydroxy-derivatives such as the clinically used naloxone, naltrexone and nalbuphine. Noroxymorphone, a semisynthetic alkaloid, is the common intermediate for the synthesis of naloxone, naltrexone, nalmefene, nalbuphine and nalfurafine.

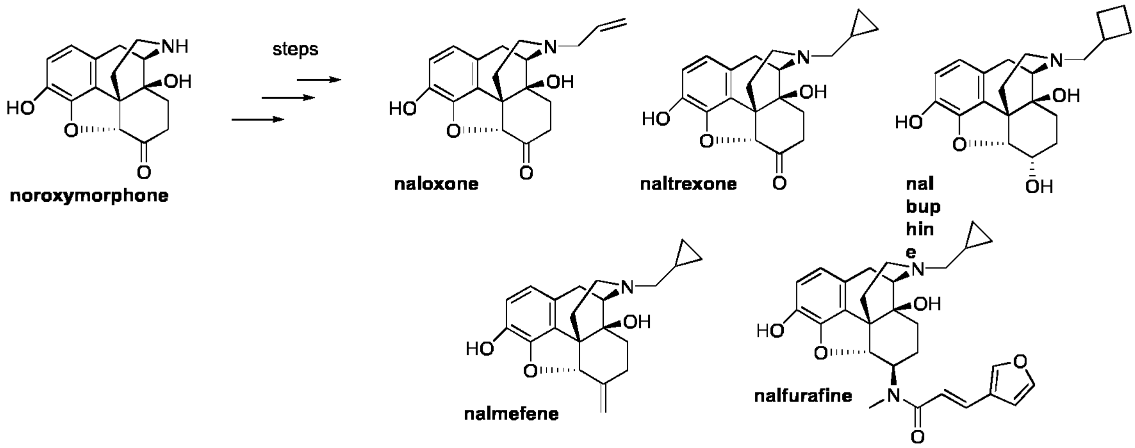

noroxymorphone naloxone naltrexone nal bup hin e nalmefene nalfurafine

The manufacture of noroxymorphone from 3,14-diacetyloxymorphone is a method described in the art as shown in scheme below:

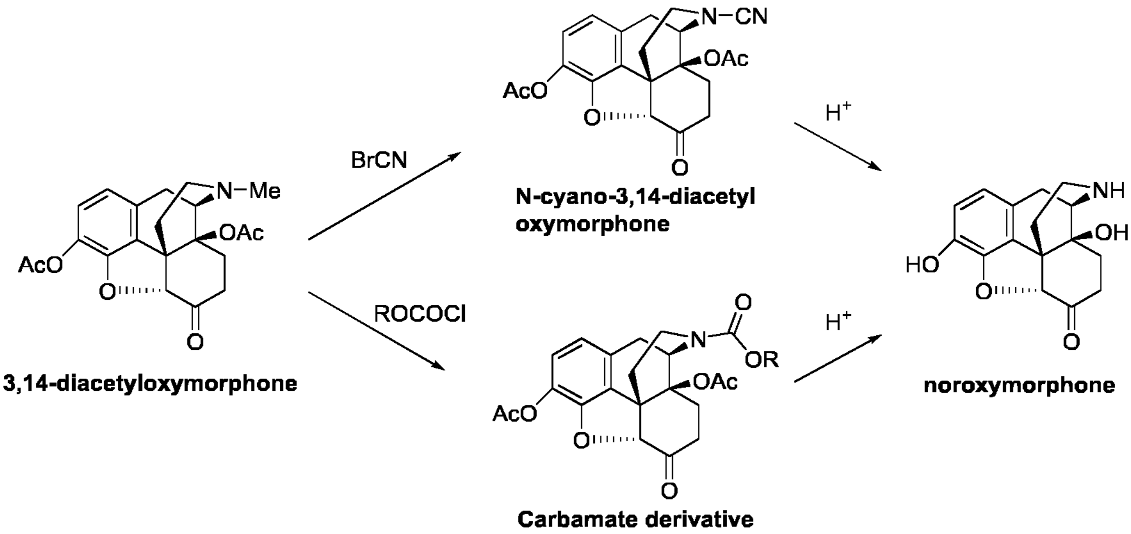


3,14-diacetyloxymorphone

N-cyano-3,14-diacetyl oxymorphone

Carbamate derivative

noroxymorphone

The N-dealkylation is typically carried out with cyanogen bromide in chloroform (e.g., US3254088, Norton et al. J. Med. Chem. 1973, 16, 556), or with chloroformates, as vinyl chloroformate (e.g., US4141897, Olofson et al., Tetrahedron Lett. 1977, 1567-1570), trichloroethyl chloroformate (e.g., GB2000137B) or ethyl chloroformate (e.g., WO2006/084412, WO2009/078989 Mallinckrodt or WO2009/122436 Sun Pharmaceuticals). Finally, acidic hydrolysis furnishes noroxymorphone.

Several processes have been disclosed in the literature for the synthesis of 3,14-diacetyloxymorphone. Most of them revolve around the use of oxycodone and/or oxymorphone as the key intermediates.

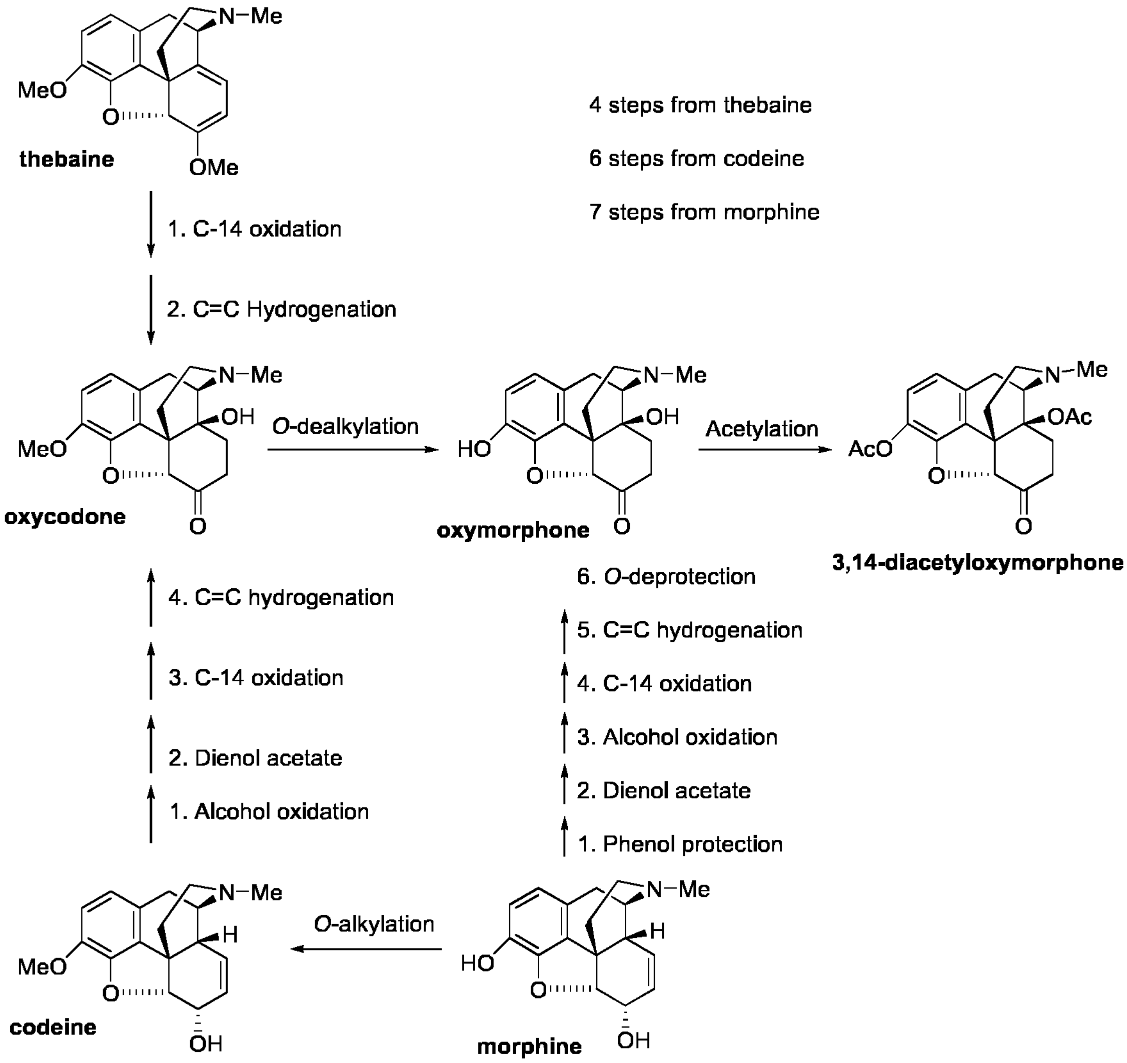


Overall, the industrial preparation of 3,14-diacetyloxymorphone through oxycodone involves several steps from naturally occurring alkaloids such as thebaine or codeine and is hampered by several problems.

The two-step synthesis of oxycodone from thebaine (e.g. EP2121698B1, EP2137190B1, EP2377866B1, Kranig et al. Arch. Pharm. Pharm. Med. Chem. 1996, 329, 325-326) was hampered by the fact that historically thebaine was a minor alkaloid found in the poppy plant, Papaver Somniferum and the medium overall yield (61-78%).

Due to the poor availability of thebaine at that time, other approaches to 3,14-diacetyloxymorphone have been tried starting from materials such as morphine (e.g. WO99/02529, Penick) and codeine (e.g. WO99/02529, Penick; Schwartz et al, J. Med. Chem. 1981, 24, 1525). Although morphine and codeine are found in greater natural abundance than thebaine, these alternative approaches involve seven or six steps, respectively.

Furthermore, a common but very challenging problem is the removal of the O-methyl group in oxycodone to oxymorphone. US2806033 claims a process for the conversion of oxycodone to oxymorphone using concentrated HBr in only 27% yield. ES465073 disclosed a procedure using pyridine hydrochloride in DMSO in 60% yield. Rice et al. (J. Org. Chem. 1998, 63, 4392) have introduced L-selectride as a reagent for the O-demethylation of oxycodone, but protection of the carbonyl group is needed and the yield is only moderate. US5071985 (Sanofi) discloses the conversion of oxycodone into oxymorphone by reaction with a very large excess of corrosive methanesulfonic acid in the presence of DL-methionine in 72% yield (see also Andre et al. Synth. Commun. 1992, 22, 2313-2327). Most of the experimental procedures for the conversion of oxycodone to oxymorphone involve the use of an excess of the expensive and corrosive BBr3 in halogenated (chloroform, chlorobenzene) solvents as disclosed in WO80/00841, WO2007/137782, Alpharma and WO2009/111162, Mallinckrodt. Rice et al. (J. Med. Chem. 1978, 21, 398) reports a yield of 76% for the demethylation reaction using $BBr_3$ in chloroform.

Overall, the current syntheses of 3,14-diacetyloxymorphone from thebaine, morphine or codeine are lengthy, low-yielding, troublesome, and expensive.

In the past, bulk supplies of oripavine, another naturally occurring alkaloid, were difficult to obtain due to its low

natural abundance in the opium poppy (Nielsen et al. Planta Med. 1983, 48, 205-206). More recently, mutant poppy strains have been developed which have a much higher content of oripavine (Millgate et al. Nature 2004, 431, 413), which has opened the possibility of its use as a bulk raw material. Oripavine is a very attractive starting material for the preparation of a number of other opiates, such as oxymorphone and derivatives thereof. Notably, oripavine lacks the O-methyl group (unlike thebaine) which obviates the need to perform an O-demethylation step during the preparation of these semi-synthetic opiates.

For example, Coop et al. (J. Org. Chem. 1996, 61, 6774 and 1998, 63, 4392-4396) reported that while oxidation of the dienol ether system of oripavine with performic acid proved somewhat problematic, m-chloroperbenzoic acid (MCPBA) gave 14-hydroxymorphinone in 62% yield. Starting from 14-hydroxymorphinone synthesized from 14-hydroxycodeinone (US 2772270), Weiss et al. had previously disclosed its reduction to oxymorphone (J. Org. Chem. 1957, 22, 1505-1508).

According to the teaching of WO2008/072018 (Johnson Matthey), the oxidation of oripavine to 14-hydroxymorphinone can also be carried out using a mixture of aqueous formic acid and 35% hydrogen peroxide. After neutralization with dilute ammonium hydroxide, 14-hydroxymorphinone is formed and filtered off. Catalytic hydrogenation of this product furnished impure oxymorphone that needs to be further purified in order to minimize the content of 14-hydroxymorphinone (an α,β-unsaturated ketone or ABUK).

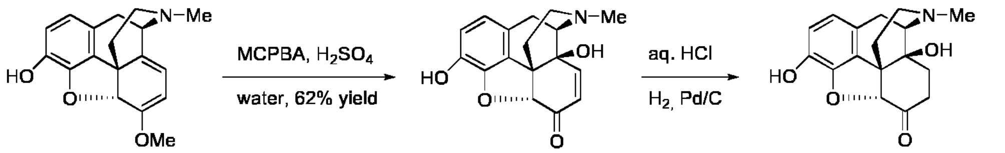

oripavine 14-hydroxymorphinone oxymorphone

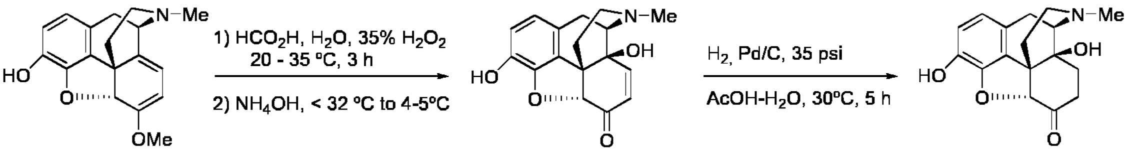

oripavine 14-hydroxymorphinone oxymorphone

Alternatively, WO2008/048711A1 (EP2073636B1, Penick Corporation) disclosed a method of preparing oxymorphone or a salt thereof comprising a single oxidizing step of oripavine to obtain 14-hydroxymorphinone, and reducing the 14-hydroxymorphinone to obtain oxymorphone, wherein the 14-hydroxymorphinone is not isolated prior to reduction. A mixture of 30% formic acid and 30% hydrogen peroxide are used for the oxidation step. The reduction is typically carried out by catalytic hydrogenation. The yields from oripavine to oxymorphone crude are in the range 87.2 to 95.2%. Purification of oxymorphone (91% yield) and addition of hydrochloric acid furnished oxymorphone HCl in 86.7% yield.

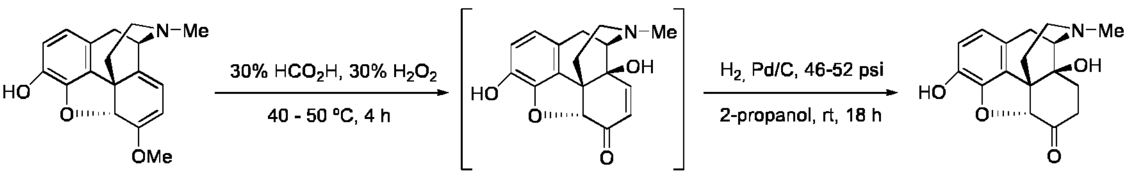

oripavine 14-hydroxymorphinone oxymorphone

Alternatively, WO2008/130553 (EP2137190B1, Mallinckrodt) and WO2013/188418 (Siegfried) have disclosed two-steps one-pot processes that involve the oxidation of oripavine or a salt thereof using peracids followed by reduction of the 14-hydroxymorphinone using hydrogen transfer conditions (e.g., $HCO_2Na$, $Et_3N$, Pd/C). WO2011/171712 (EP2377866B1, Siegfried) has disclosed several continuous flow reactor processes from oripavine to oxymorphone, including some one-pot reactions.

Of importance, the synthesis of oxymorphone from oripavine is not problem-free. Some of the issues related to this approach are:

a) It is known that the oxidation of oripavine may be troublesome. For example, it may furnish mixtures of 14-hydroxymorphinone and its corresponding N-oxide derivative (e.g., WO2008/118654, Mallinckrodt). In fact, it is possible to selectively oxidize oripavine to its N-oxide hydrochloride without affecting the dienol methyl ether subunit

(Kok et al. Org. Biomol. Chem. 2011, 9, 1008-1011) or to carry out directly the oxidation of oripavine to 14-hydroxymorphinone-N-oxide (WO2005/028483, GSK).

b) The catalytic hydrogenation of 14-hydroxymorphinone HCl in aqueous acetic acid may furnish some amounts of 6-hydroxy by-product, produced by the over-reduction of the desired product (Kok et al. RSC Advances 2012, 2, 11318-11325).

c) In the common practice in industry to perform the two-step sequence in one-pot, without isolation of the 14-hydroxymorphinone, the presence of any excess of oxidizing agent has been reported to result in the formation of 1,1'-dimer by-products. These dimers are more insoluble than oxymorphone, and are therefore difficult to remove by recrystallization (see WO 2008/118654, EP2125824B1 Mallinckrodt). These impurities may be carried over during the synthesis producing 2,2'-binaloxone or pseudonaltrexone, typical impurities of naloxone and naltrexone, respectively.

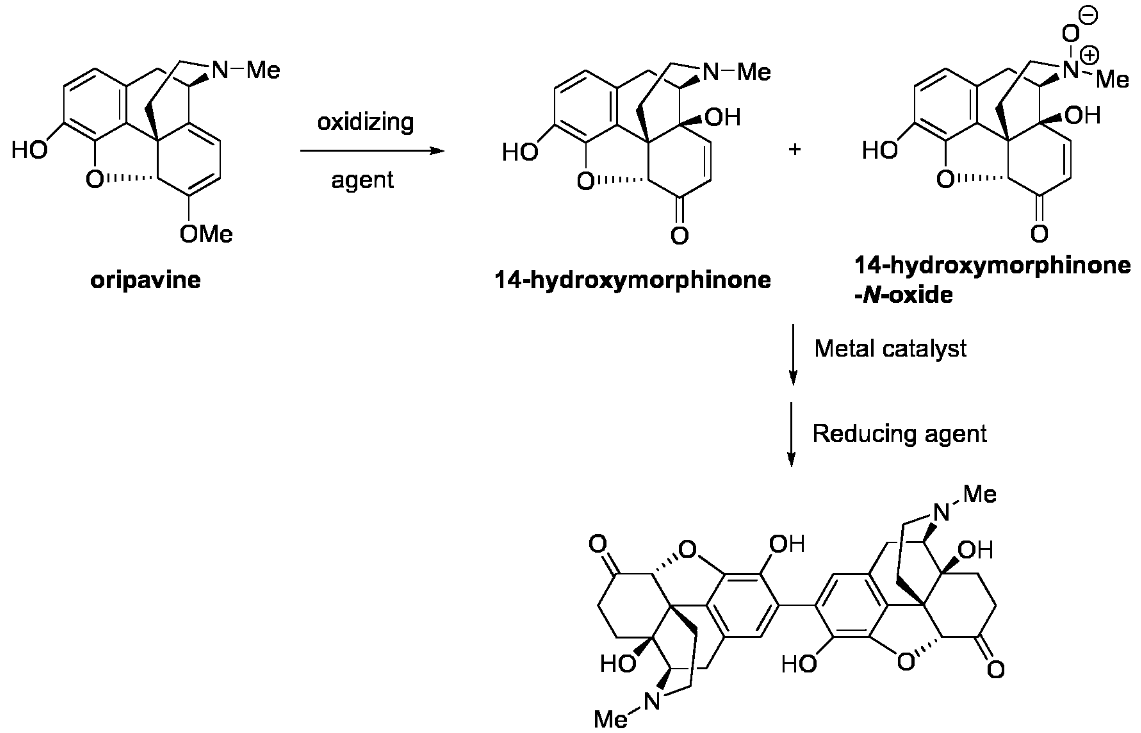


oripavine 14-hydroxymorphinone 14-hydroxymorphinone -*N*-oxide

d) During the oxidation of oripavine to 14-hydroxymorphinone and reduction to oxymorphone certain byproducts can be formed, e.g., 8α-hydroxyoxymorphone (also referred to as 8α,14-dihydroxydihydromorphinone) and 8β-hydroxyoxymorphone (also referred to as 8β,14-dihydroxydihydromorphinone) due to a reaction of 14-hydroxymorphinone with water present in the reaction mixture. These by-products can be further converted to 14-hydroxymorphinone when acidic medium is used (WO 2014/013313, Rhodes). It has further been found that long holding periods of solutions of oxymorphone can lead to increased levels of 14-hydroxymorphinone. This effect is especially showing at higher temperatures, in particular at the boling point of the solvent.

The presence of small amounts of 14-hydroxymorphinone or of the aforementioned α,β-unsaturated ketone precursors (e.g., 8-hydroxyoxymorphone) is of concern. 14-hydroxymorphinone belongs to a class of compounds known as α,β-unsaturated ketones (ABUKs). These compounds contain a substructural component (the α,β-unsaturated ketone unit) which produces a structure-activity relationship alert for genotoxicity.

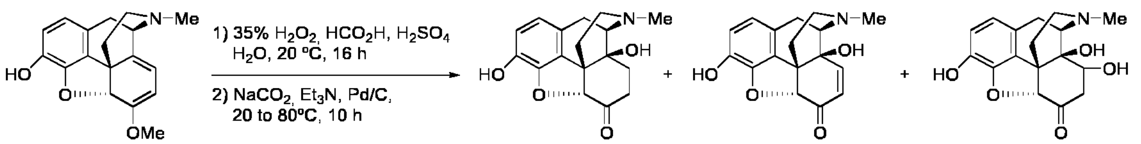


oripavine oxymorphone 14-hydroxymorphinone 8-hydroxyoxymorphone

As expected, the amount of these byproducts is higher when the synthesis of oxymorphone is carried out in a one-pot process from oripavine. For example, according to the teaching of WO2013/188418, the oxymorphone HCl obtained from oripavine without isolating 14-hydroxymorphinone and oxymorphone base crude contained 1703 ppm of 8,14-dihydroxydihydromorphinone.

The resulting oxymorphone, and its derivatives, thus have to be subjected to one or more additional purification steps (e.g. further reduction as in WO2008/072018 Johnson Matthey, recrystallizations, etc.) to reduce the amount of ABUKs below the limits set for the regulatory authorities (< 100 ppm), increasing the production costs. Moreover, when submitted to further reduction in order to minimize ABUKs contents, small amounts of 6-noroxymorphols have been detected (WO2015/015147, Mallinckrodt).

Also, it is known that 14-hydroxymorphinone undergoes hydration to 8-hydroxyoxymorphone in aqueous basic medium and that 8-hydroxyoxymorphone dehydrates back to 14-hydroxymorphinone in acidic medium (Weiss, J. Org. Chem. 1957, 22, 1505-1508; see also WO2013/188418). Also, it is believed that under aqueous acidic conditions an equilibrium occurs between 14-hydroxymorphinone and 8-hydroxyoxymorphone (see p. 5, WO2015/015147 Johnson Matthey)

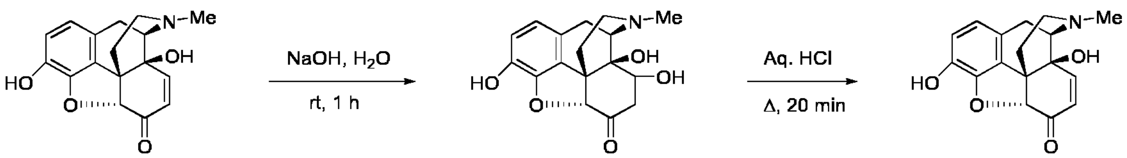


**14-hydroxymorphinone** **8-hydroxyoxymorphone** **14-hydroxymorphinone**

In other words: if oripavine is converted to oxymorphone without isolating 14-hydroxymorphinone, dimers may be an issue, as is partial hydrogenation and partial N-oxidation; if 14-hydroxymorphinone is isolated, there is one step more and there are some stability issues of the product that may have impact in the ABUKs content of the final APIs.

Overall, the current syntheses of oxymorphone and thus of 3,14-diacetyloxymorphone from oripavine are troublesome and expensive. There exists therefore a need for an improved method of synthesis of 3,14-diacetyloxymorphone, noroxymorphone and other morphinan derivatives that provides a significant yield improvement, as well as a higher quality with a minor amount of ABUKs contents, preferably without the intermediacy of 14-hydroxymorphinone.

From 3,14-diacetyloxymorphone two classical synthetic steps lead to noroxymorphone. The *N*-dealkylation of 3,14-diacetyloxymorphone is typically carried out with cyanogen bromide in chloroform (e.g., US3254088, Norton et al. J. Med. Chem. 1973, 16, 556), or with chloroformates, as vinyl chloroformate (e.g., US4141897, Olofson et al., Tetrahedron Lett. 1977, 1567-1570), trichloroethyl chloroformate (e.g., GB2000137B) or ethyl chloroformate (e.g., WO2006/084412, WO2009/078989 Mallinckrodt or WO2009/122436 Sun Pharmaceuticals). Finally, acidic hydrolysis furnishes noroxymorphone.

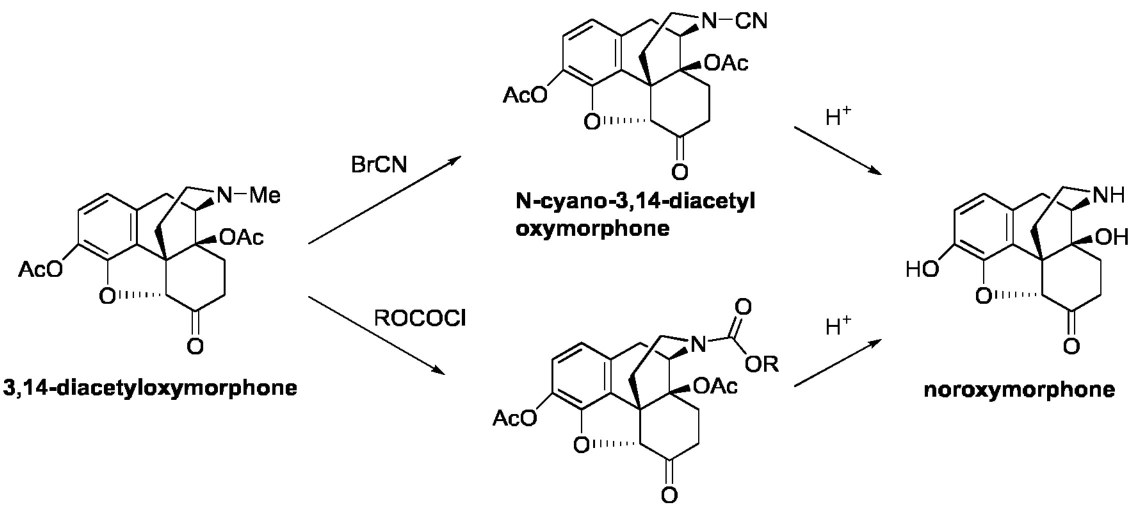


**N-cyano-3,14-diacetyl oxymorphone**

**3,14-diacetyloxymorphone**

**noroxymorphone**

**Carbamate derivative**

Although, these routes for preparing noroxymorphone from diacetyloxymorphone are known to the expert in the field, they have until now only been described in conditions which are not unproblematic. For example, they require the use of very toxic chlorinated solvents (chloroform, 1,2-dichloroethane, chlorobenzene), the use of a very large excess of toxic cyanogen bromide or the use of expensive and toxic chloroformates, typically used in large excess (e.g.,

WO2006/084412, WO2009/122436).

There exists therefore a need for an improved method of synthesis of 3,14-diacetyloxymorphone, preferably without the need of synthesizing oxycodone or oxymorphone, that provides a significant yield improvement, as well as the option of a one pot synthesis wherein the product is formed in a single reaction vessel without isolating the intermediate, and with a minor amount of ABUKs contents.

## Summary of the invention

The present inventors have designed a process to manufacture 3,14-diacetyloxymorphone involvings four synthetic transformations from oripavine, wherein only two intermediate products need to be isolated, since two sets of reactions may be carried out as one-pot reactions.

Regarding the previous art, the process of the invention has the following advantages:

a) oripavine is selected as the starting material, avoiding the troublesome O-demethylation step involved in the prior art processes starting from thebaine or codeine,

b) 14-hydroxymorphinone is not involved in the synthesis, thus avoiding its stability issues,

c) the formation of 1,1'-dimers is minimized. This is achieved through the acetyl protection as well as because the oxidation and the hydrogenation reactions are carried out in separate steps,

d) the hydrogenation of the 3-acetyloxymorphinone works very well (>95% yield), in surprising contrast with the hydrogenation of 3,14-diacetylroxymorphinone, that furnishes 3,14-diacetyloxymorphone in medium yield (70%).

e) it has been found that when using 3,14-diacetyloxymorphone obtained by this new route to obtain noroxymorphone the level of 6-hydroxy derivatives is very low (often lower than 0.03% by weight).

f) it has been found that when using 3,14-diacetyloxymorphone obtained by this new route to obtain noroxymorphone the level of $\alpha,\beta$-unsaturated ketones (ABUKs) in the final noroxymorphone is <1000 ppm (without purification) or <500 (via sulphate).

3,14-Diacetyloxymorphone obtained by this new route has been successfully converted into noroxymorphone and then into naloxone, naltrexone or nalbuphine.

## Detailed description of the invention

In a first aspect the present invention relates to a process (shown in Scheme 1) for preparing 3,14-diacetyloxymorphone (I) comprising the steps of:

a) reacting oripavine (II) with an acetylating agent to yield compound (III),

b) reacting compound (III) with an oxidizing agent to yield compound (IV),

c) reacting compound (IV) with a hydrogenating agent to yield compound (V),

d) reacting compound (V) with an acetylating agent to yield compound (I),

Scheme 1

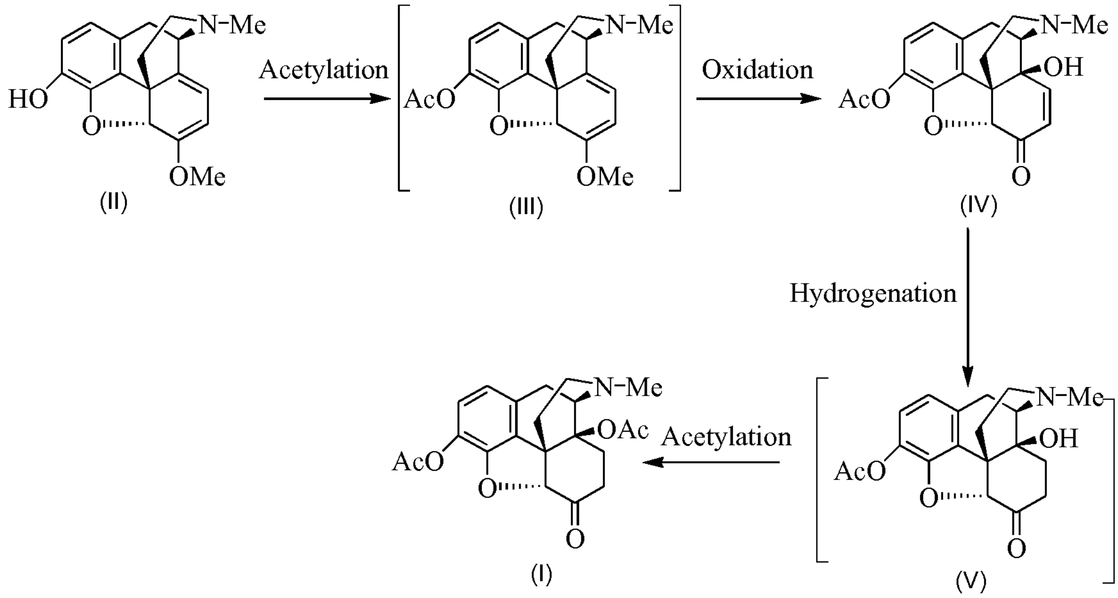


Noroxymorphone (VIII) can also be obtained from orapavine (II) in a process wherein 3,14-diacetyloxymorphone is prepared as described above and then converted into noroxymorphone as shown in Scheme 2

Scheme 2

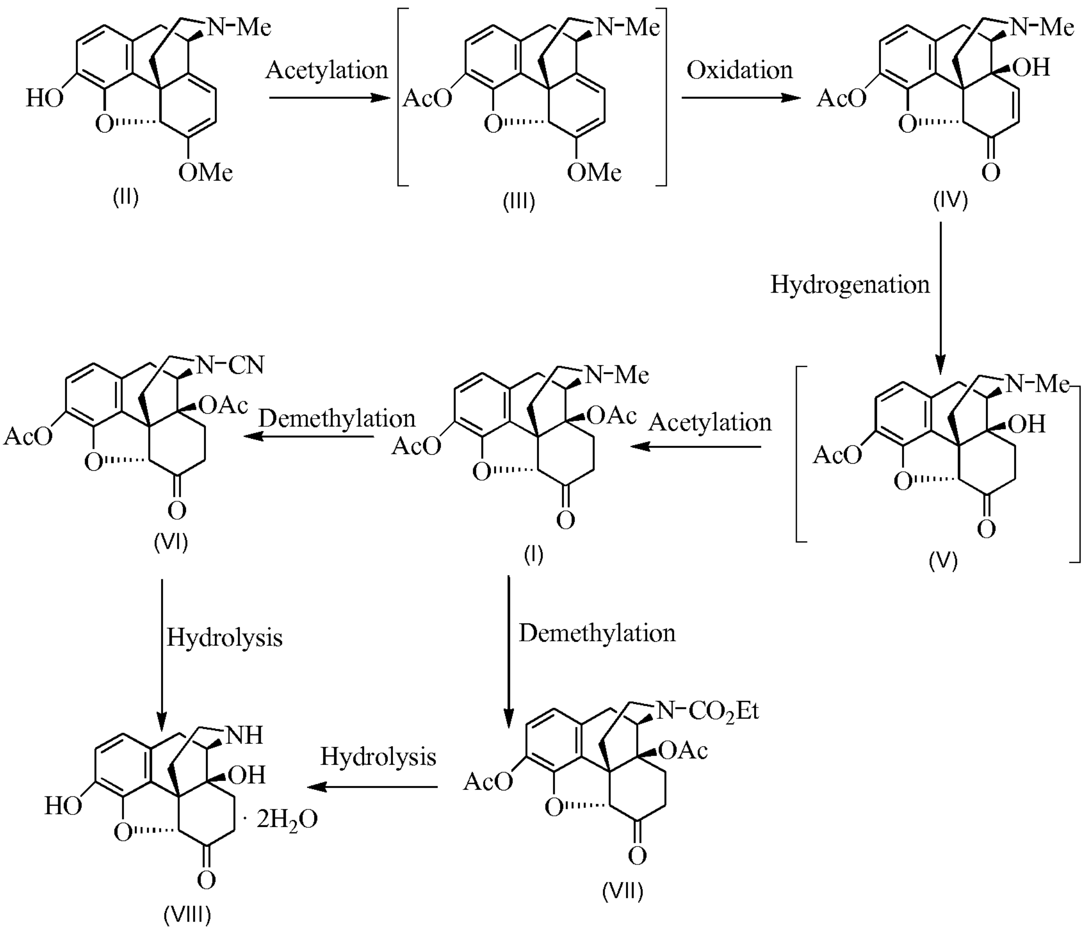


(II) (III) (IV) (V) (I) (VI) (VII) (VIII)

Thus, in a second aspect of the present invention a process (shown in Scheme 2) for the preparation of compound of formula (VIII) starting from compound of formula (II) comprising the following steps:

a) reacting oripavine (II) with an acetylating agent to yield compound (III),

b) reacting compound (III) with an oxidizing agent to yield compound (IV),

c) reacting compound (IV) with a hydrogenating agent to yield compound (V),

d) reacting compound (V) with an acetylating agent to yield compound (I),

e) reacting compound (I) with a demethylating agent to yield either compound (VI) or compound (VII), and

f) reacting either compound (VI) or compound (VII) with a hydrolising agent to yield noroxymorphone (VIII)

In a third aspect of the present invention noroxymorphone (VIII) which has been obtained according to the second aspect of the invention is used for the preparation of naloxone, naltrexone, nalbuphine or nalmefene.

For comparative purposes the inventors have also carried out the synthesis of 3,14-diacetyloxymorphone (I) from oripavine (II) following an alternative route (shown in scheme 3 and in Comparative example 1) not falling within the scope of the present invention.

This alternative route employs the same type of reactions used in the route of scheme 2 according to the invention; i.e. oxidation of the 1-methoxy-cyclohexa-1,3-diene ring to a 4-hydroxy-cyclohex-2-enone ring, acetylation of the free hydroxyl groups, and hydrogenation of the 4-hydroxy-cyclohex-2-enone ring to a 4-hydroxy-cyclohexanone ring to go from compound (IX) to compound (I). However, the inventors have found that in this alternative the catalytic hydrogenation of the diacetate, compound of formula (X), furnished a final compound of formula (I) having substantial levels of several impurities.

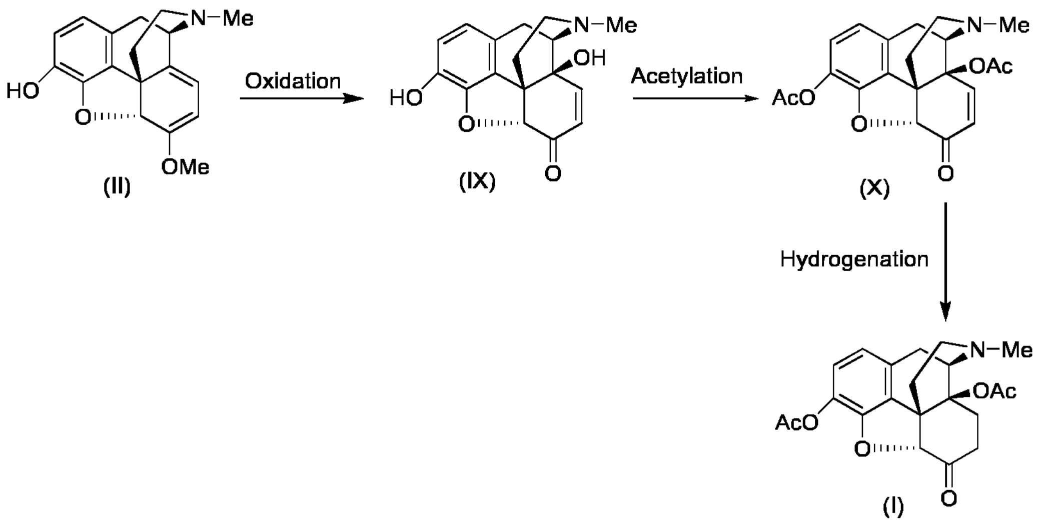


Scheme 3

In an embodiment of the present invention step b) is carried out after step a) without isolating or purifying product (III).

In another embodiment of the present invention step d) is carried out after step c) without isolating or purifying product (V).

In still another embodiment of the present invention the product (IV) is isolated from the reaction mixture resulting from step b) before carrying out step c).

In an embodiment of the present invention the acetylating agents used in steps a) and d) may be the same of different and are preferably selected from the group consisting of acetyl halides and acetic anhydride. Examples of suitable acetylating agents are acetyl chloride, acetyl bromide and acetic anhydride, in particular acetic anhydride.

In an embodiment of the present invention the oxidizing agent used in step b) is an organic peracid or a precursor thereof. In another embodiment of the invention the organic peracid or the precursor thereof is selected from the group consisting of peracetic acid, mixture of $H_2O_2$ and acetic acid, formic acid, mixture of $H_2O_2$ and formic acid and meta-chloroperbenzoic acid, preferably a mixture of $H_2O_2$ and acetic acid.

In an embodiment of the present invention the hydrogenating agent used in step c) is hydrogen gas in the presence of a metal catalyst such as Pd, Pt, Ni (optionally in the presence of active carbon) or another source of hydrogen under transfer conditions selected from the group consisting of sodium formate and triethylamine in the presence of a metal catalyst such as Pd/C.

In an embodiment of the present invention product (I) may be advantageously purified by recrystallization from 2-methyltetrahydrofuran (2-MeTHF).

In an embodiment of the second aspect of present invention the demethylating agent used in step e) to convert compound (I) to compound (VI) is cyanogen bromide, preferably in chloroform or acetonitrile, most preferably in acetonitrile. This process is described in US 3,254,088 which is incorporated by reference.

In an embodiment of the second aspect of present invention the demethylating agent used in step e) to convert compound (I) to compound (VII) is a chloroformate, such as ethyl chloroformate or vinyl chloroformate. This process is described in WO 2006/084412, WO 2009/078989 and WO 2009/122436 which are incorporated by reference.

In an embodiment of the second aspect of the present invention the hydrolising agent used in step f) to convert compound (VI) or compound (VII) to noroxymorphone (VIII) is an acid having a pHa lower than 5 such as sulfuric acid.

Process for the determination of $\alpha,\beta$-unsaturated ketones (ABUK) in the products and intermediates of the invention.

**General HPLC Method (used to follow reactions and to check the purity of the products)**

Column: Hypersil ODS2. 200 x 4.6 mm, 5 μm from Thermo Scientific.

Mobil Phase: Buffer diethylamine phosphate pH 6.5 and acetonitrile.

Buffer: diethylamine phosphate pH 6.5: (0.86 g $H_3PO_4$ 85%, 1 liter of water and adjust pH at 6.5 with diethyl amine)

Gradient

| Time (minutes) | % Buffer | % Acetonitrile |
|---|---|---|
| 0 | 95 | 5 |
| 5 | 90 | 10 |
| 15 | 50 | 50 |
| 25 | 30 | 70 |
| 30 | 95 | 5 |
| 33 | 95 | 5 |

Temperature: 30° C

Flow: 1 ml/min

Detector: 230nm

Injection Volume: 10 μl

Injection system: automatic.

Preparation of Samples

The samples are dissolved in acetonitrile or 0.1 M HCl.

IPC: about 100 mg of sample in 50 mL

Solid samples: about 25 mg in 50 mL

*Retention times (minutes)*

| product | ODS2 (tr) |
|---|---|
| Oripavine (II) | 11.8 |
| 3-Acetyloripavine (III) | 15.8 |
| 3-acetyloxymorphinone (IV) | 14.1 |
| 3-acetyloxymorphone (V) | 12.2 |
| 3,14-diacetyloxymorphone (I) | 16.6 |
| N-Cyano-3,14-diacetyloxymorphone (VI) | 15.6 |
| Noroxymorphone (VIII) | 5.4 |
| N-carbamoyl-3,14-diacetyloxymorphone (VII) | 18.1 |
| 14-hydroxymorphinone | 10.0 |
| 3,14-diacetyloxymorphinone | 16.6 |
| N-Cyano-3,14-diacetyloxymorphinone | 16.6 |
| Nor-14-hydroxymorphinone | 5.5 |
| Naloxone | 16.0 |

(continued)

| product | ODS2 (tr) |
|---|---|
| Naltrexone | 13.6 |
| Nalbuphine | 15.6 |

This assay allows detecting the course of the different reactions and the presence and amount of the different intermediates, final products and by-products.

It forms the basis for the calculation of the value "HPLC purity %". This value, calculates the % of the product of interest in a given mixture by comparing the area of the peak corresponding to the product with the areas of all the peaks identified in the chromatogram.

It also forms the basis for the calculation of the HPLC assay %". This value is calculated from the relation between the area and the weight of the standard and the area and the weight of the sample.

**HPLC Method for ABUK determination**

Mobile phase A: Weigh 0.777(±0.03) g of Ammonium acetate into a suitable mobile phase bottle, dissolve with 950ml of deionised water, add 25ml of Acetonitrile and 25ml of MeOH to the container. Mix well and degas.

Mobile phase B: Weigh 0.777(±0.03) g of Ammonium acetate into a suitable mobile phase bottle, dissolve with 100ml of deionised water, add 450ml of Acetonitrile and 450ml of MeOH to the container. Mix well and degas.

Operating conditions:

Column: ODS 5μm, 4.6X250mm.
Column temperature: 50 °C.
Injection volume: 10μl.
Detection: 220nm.
Flow rate: 1 ml/min.
Analysis time: 40 min.
Run time: 48min.

Linear gradient conditions:

| Time minutes | % MPA | % MP B |
|---|---|---|
| 0 | 100 | 0 |
| 40 | 0 | 100 |
| 45 | 100 | 0 |
| 48 | 100 | 0 |

*Retention times (minutes)*

| Product | C18 (tr) |
|---|---|
| 3,14-diacetyloxymorphone | 27.5 |
| 3,14-diacetyloxymorphinone (ABUK) | 26.7 |

*Preparation of Samples*

Standard of ABUK

Dissolve 15 mg of 3,14-diacetyloxymorphinone (ABUK) in 50 ml of acetonitrile and dilute 1 mL of this solution to 50 mL with acetonitrile.

Test Solution

Dissolve about 35 mg of 3,14-diacetyloxymorphone.

*Calculations*

$$\% \text{ ABUK in sample} = (A_{sample}/A_{std}) \text{ x } (C_{std}/C_{sample}) \text{ x } 100$$

Where:

$A_{sample}$: mean the area of the peak due to ABUK in the chromatogram obtained with test solution.

$A_{stp}$: mean the area of the peak due to ABUK in the chromatogram obtained with standard solution.

$C_{sample}$: mean the concentration in mg/mL of the test solution.

$C_{std}$: mean the concentration in mg/mL of the standard.

**HPLC Method for ABUK determination in noroxymorphone**

Mobile phase

Prepare a solution as follows: dissolve 1.17 g of sodium octanesulphonate in 1000 mL of water, adjust pH to 2.0 with 50% v/v solution of phosphoric acid.

MP A: 20 mL acetonitrile, 40 mL tetrahydrofuran and 940 mL of above solution

MP B: 170 mL acetonitrile, 40 mL tetrahydrofuran and 790 mL of above solution

Operating conditions:

Column: Kromasil C8 (or equivalent), 5μm, 4.0X125mm.

Column temperature: 40 °C.

Injection volume: 20μl.

Detection: 230nm.

Flow rate: 1.5 ml/min.

Linear gradient conditions:

| Time minutes | % MP A | % MP B |
|---|---|---|
| 0 | 100 | 0 |
| 40 | 0 | 100 |
| 50 | 0 | 100 |
| 55 | 100 | 0 |
| 65 | 100 | 0 |

*Retention times (minutes)*

| Product | Kromasil (tr) |
|---|---|
| Noroxymorphone | 10.5 |
| Nor-14-hydroxymorphinone | 12.2 |
| 8-hydroxy-oxymorphone | 8.4 |

*Preparation of Samples*

Standard 1 ppm

Dissolve 10 mg of mixture 1:1 of the NHM and 8-OH in 100 ml of 0.1M HCl and dilute 2 mL of this solution to 100 mL with 0.1M HCl

Considerer than the sample is a mixture 1:1 of the NHM and 8-OH, then the final solution will be 1 ppm for each compound.

Test Solution

Dissolve about 50 - 150 mg of norxymorphone in 50 mL of 0.1M HCl.
The solution is about 1000 to 3000 ppm.

*Calculations*

$$\text{ppm in sample} = (A_{sample}/A_{std}) \times (C_{std}/C_{sample}) \times 1000000$$

Where:

$A_{sample}$: mean the area of the peak due to any impurity in the chromatogram obtained with test solution.

$A_{stp}$: mean the area of the peak due to any impurity in the chromatogram obtained with standard solution.

$C_{sample}$: mean the concentration in ppm of the test solution.

$C_{std}$: mean the concentration in ppm of the standard.

**COMPARATIVE EXAMPLES:**

Comparative Example 1

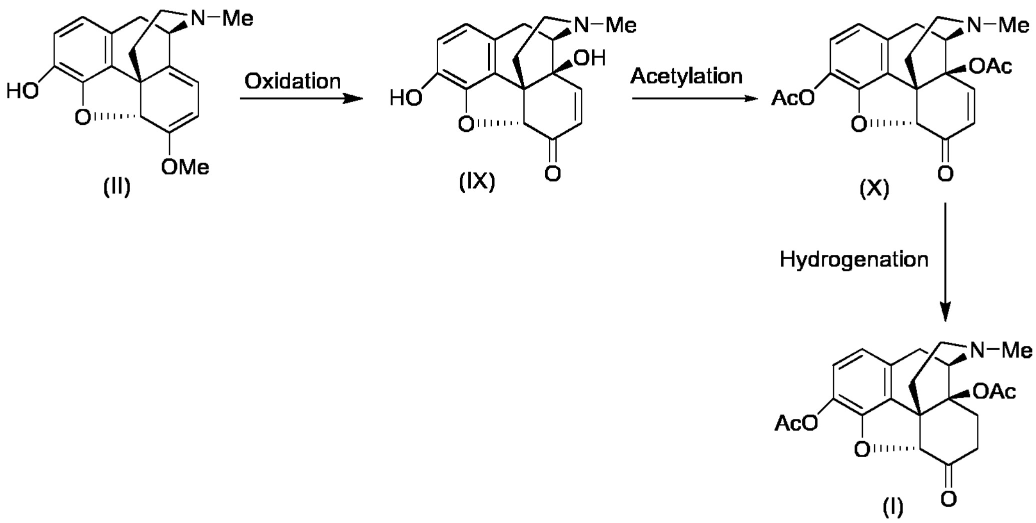


(II) (IX) (X) (I)

Step 1. Oxidation of oripavine to 14-hydroxymorphinone (IX)

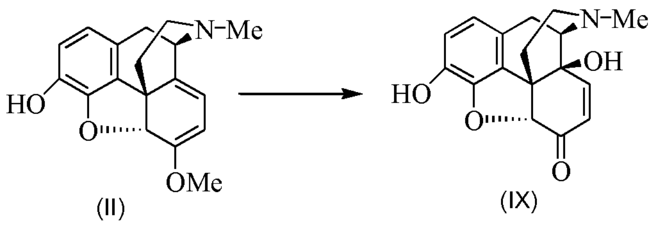

(II) (IX)

Oripavine (II) (80 g, 0.269 mol) was added in 20 minutes to a solution of water (42.4 g) and formic acid (220.8 g) at 20 - 25 °C. The mixture was stirred at 20 - 25 °C until complete dissolution (15 - 20 minutes). 30% Hydrogen peroxide (33.6 g) was added dropwise in one hour at about 20 °C and the solution was stirred for 16 h at 15 - 20 °C. The reaction was cooled at 5 - 10 °C and then ammonia (300 mL) was added until pH 8.5, 14-hydromorphinone was precipitated; the suspension was stirred for 4 hours at 2 ° C and the solid was filtered and washed with water (55 mL). After drying, 81.4 g of light brown solid was obtained. HPLC assay: 85%. HPLC purity: 99%. The yield was 86%

Step 2. Acetylation of 14-hydroxymorphinone to 3,14-diacetyloxymorphinone (X)

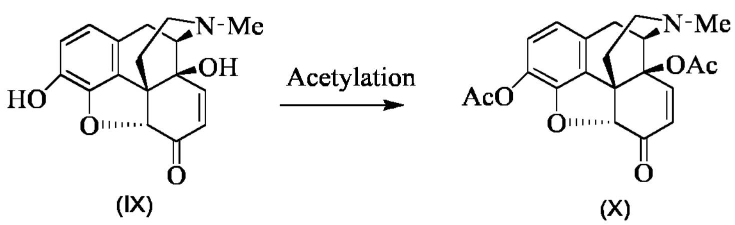


(IX) (X)

14-hydromorphinone (IX) (80 g, 85% assay. 0.227 mol) was suspended in toluene (280 mL) and the suspension was heated at reflux. Water and formic acid was distilled azeotropically (Dean-Stark, 11 mL was distilled). After that,

acetic anhydride (90 g, 0.882 mol) was added and the solution was stirred for about 10 hours at reflux. The solution was cooled at rt and the solvent was evaporated; acetic acid and acetic anhydride was distilled azeotropically with fresh toluene. 81 g of yellow solid was obtained after evaporation at dryness. HPLC assay: 98%. HPLC purity: 98%. The yield was 91%

Step 3. Process of acetylation of 14-hydroxymorphinone (IX) followed by hydrogenation.

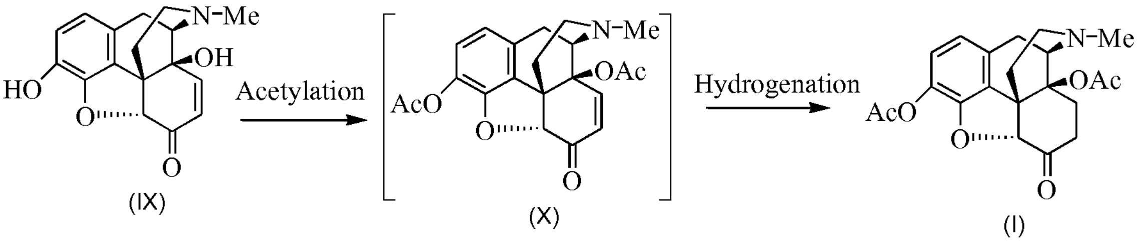


(IX) (X) (I)

Preparation of 3,14-diacetyloxymorphinone (X)

14-hydromorphinone (IX) (23 g, 90% assay. 0.069 mol) was suspended in toluene (200 g) and the suspension was heated at reflux. Acetic anhydride (37.5 g, 0.367 mol) was added and the solution was stirred for about 6 hours at reflux. The solution was cooled at rt and one 1/10 of the same was departed.

The solvent was evaporated and acetic acid and acetic anhydride was distilled azeotropically with fresh toluene. 20.6 g of yellow solid (3,14-diacetyloxymorphinone, X) was obtained after evaporation at dryness. HPLC assay: 99%. HPLC purity: 99%. The yield was 86%.

*Hydrogenation alternative 1(one-pot)*

To 1/10 of the solution of the acetylation reaction (30.9 g, 3 g of 3,14-diacetyloxymorphinone) was added 0.3 g of Pd/C (50% wet) and then the reaction was hydrogenated at atmospheric pressure giving 3,14-diacetyloxymorphone (I) with 52% yield.

*Hydrogenation alternative 2 (with isolation of 3,14-diacetyloxymorphinone)*

2 g of isolated 3,14-diacetyloxymorphinone (5.2 mmol) was dissolved in THF (30 g) at rt and then, 0.3 g of Pd/C (50% wet) and triethylamine (0.11 g, 1.09 mmol) were added. The reaction was hydrogenated at atmospheric pressure giving 3,14-diacetyloxymorphone (I) with 87% yield.

*Hydrogenation alternative 3 (with isolation of 3,14-diacetyloxymorphinone followed by purification in 2-methyltetrahydrofuran)*

41 g of 3,14-diacetyloxymorphinone (X) (0.105 mol, 98% assay) was dissolved in THF (360 g) at rt and then, 3.28 g of Pd/C (50% wet) and triethylamine (2.13 g, 0.021 mmol) were added. The reaction was hydrogenated at 65 psi at 22 °C for about 10 hours. The catalyst was filter over celite, washed with THF and the solvent was evaporated in vacuo giving 40.2 of crude 3,14-diacetyloxymorphone (HPLC purity 87% and HPLC assay 85%, 83% yield). The product was purified by crystallization in 2-methyltetrahydrofuran affording 28.5 g of pure product (HPLC purity 98% and HPLC assay 99%). Yield: 70.5%.

Comparative example 2

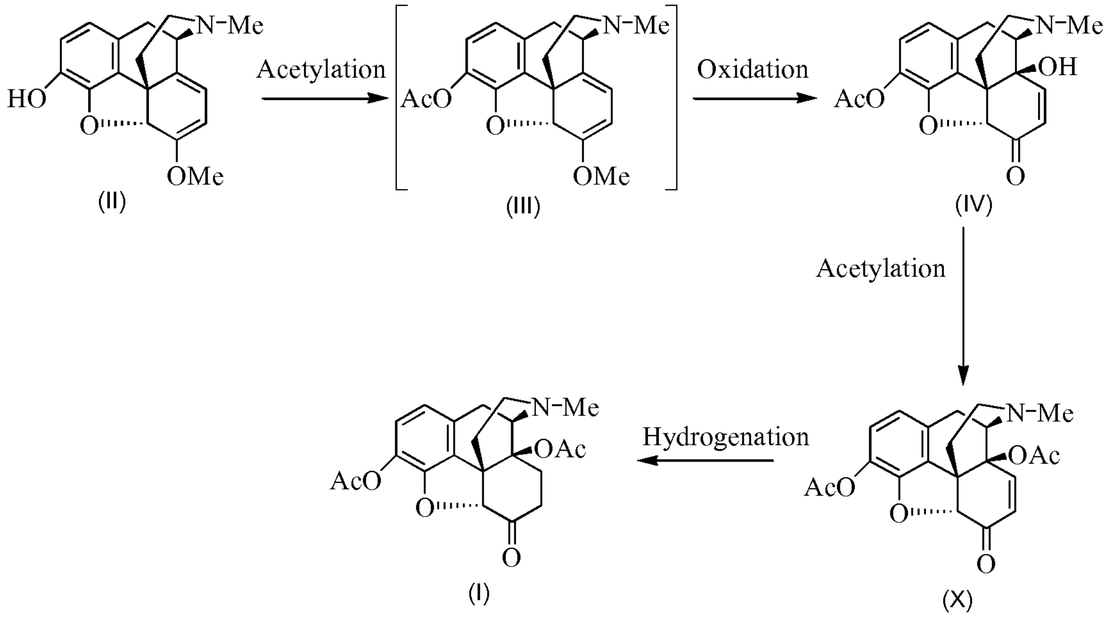


(II) (III) (IV) (I) (X)

Steps 1 and 2. Acetylation of oripavine followed by oxidation (one-pot).

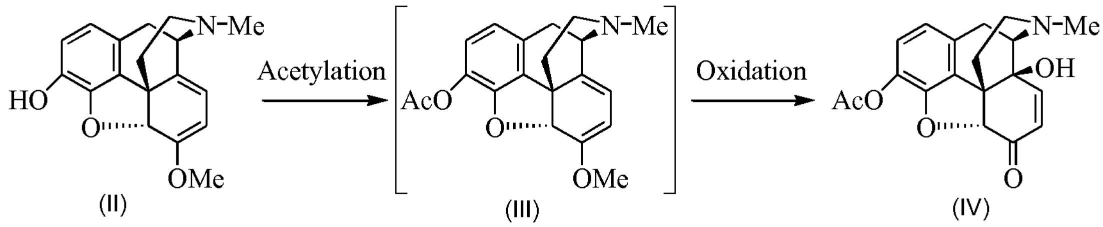


(II) (III) (IV)

Oripavine (10.0 g, 0.0336 mol) was added portion-wise to acetic anhydride (21.6 g, 0.212 mol) under an ice bath in 10 minutes and the resulting suspension was stirred for 2 hours at room temperature. Water (4.0 g) was added (ice bath) and the mixture was stirred at ambient temperature for 2 additional hours. Hydrogen peroxide (4.0 g, 0.0352 mol) was added dropwise under an ice bath in 80 minutes and the reaction was left stirring overnight at room temperature. The resulting dark solution was diluted with ethyl acetate and water and the pH was brought to 8.5 with 20% $Na_2CO_3$ aqueous solution. The suspension was filtered and the obtained solid was rinsed with water and dried affording (IV) (9.08 g, 77% yield considering 97% HPLC assay) as a brownish solid with 99% purity.

Step 3. Acetylation of 3-acetyl-14-hydroxymorphinone (IV) to 3,14-diacetyloxymorphinone (X).

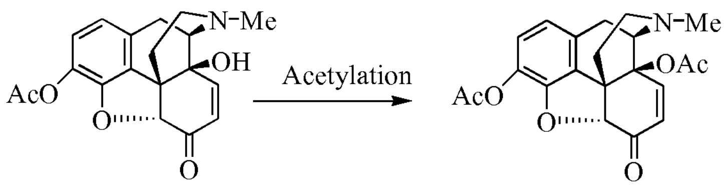


(IV) (X)

3-acetyl-14-hydroxymorphinone (IV) (4.5 g, 94.6% assay. 0.0124 mol) was suspended in toluene (45 mL) and acetic anhydride (2.35 g, 0.0231 mol) was added. The suspension was stirred for about 2 hours at 100 °C and the solution was cooled at rt, a solid was precipitated. The solid was filtered and washed with toluene; 3 g of beige solid

was obtained after drying. HPLC assay: 99%. HPLC purity: 99.6%. The yield was 62.5 %.

The mother liquors ware evaporated at dryness affording 1.9 g of additional product (as beige solid). HPLC assay: 96%. HPLC purity: 96%. The yield of this recovery was 38.4%

Step 4.

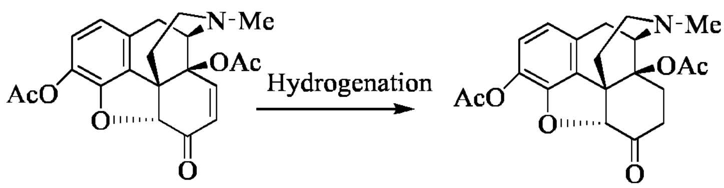


**(X)** **(I)**

3,14-diacetyloxymorphinone (X) (3.0 g, 7.7 mmol, 98% assay) was dissolved in THF (30 g) at room temperature and then, Pd/C (0.3 g, 50% wet) and triethylamine (0.16 g, 1.53 mmol) were added. The reaction was hydrogenated at atmospheric pressure at 20 °C for about 20 hours. The catalyst was filtered over celite, washed with THF and the solvent was evaporated in vacuo giving 3.0 g of crude 3,14-diacetyloxymorphone (HPLC purity 87% and HPLC assay 83%, 84% yield).

Comparative example 3.

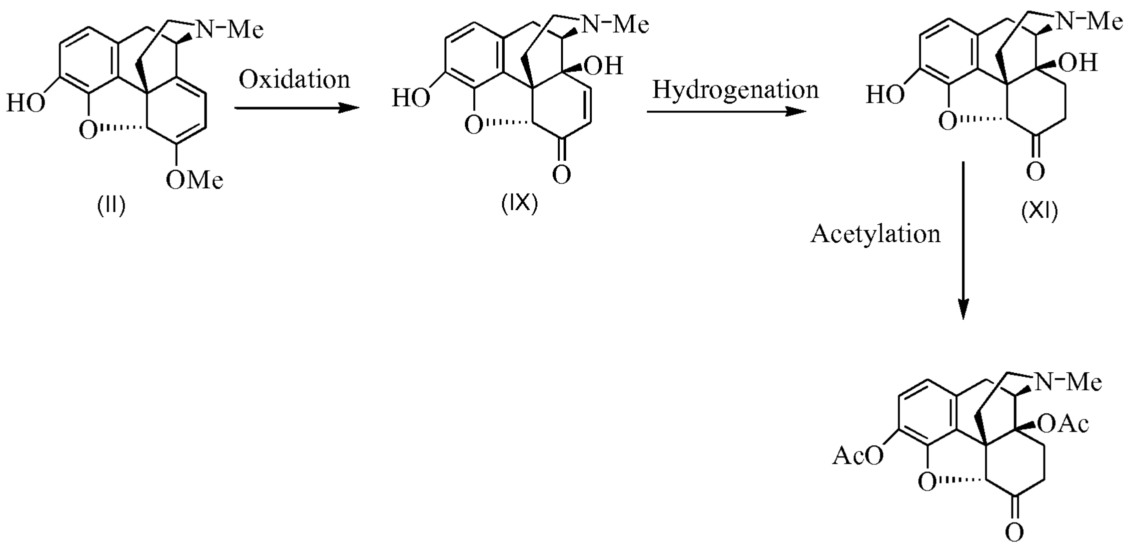


(II) (IX) (XI) (I)

Steps 1 and 2. Oxidation of oripavine (II) followed by hydrogenation of 14-hydroxymorphinone (IX) to oxymorphone (XI).

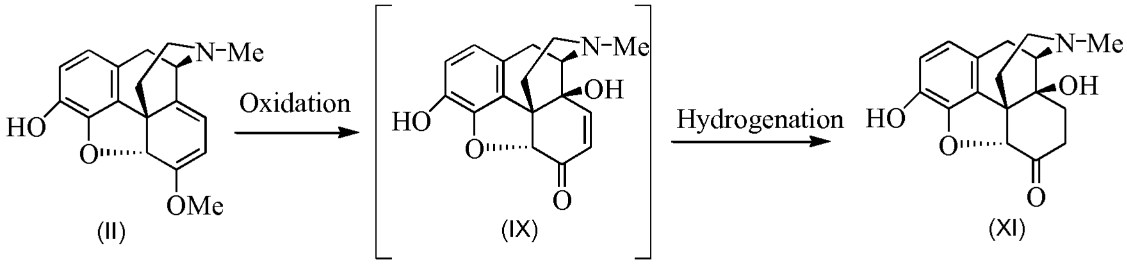


(II) (IX) (XI)

Alternative a)

Oripavine (80 g, 0.269 mol) was added in 20 minutes to a solution of water (42.4 g) and formic acid (220.8 g) at 20 - 25 °C. The mixture was stirred at 20 - 25 °C until complete dissolution (15 - 20 minutes). 30% Hydrogen peroxide (33.6 g) was added dropwise in one hour at about 20 °C and the solution was stirred for 16 h at 15 - 20 °C. The reaction was divided in two parts. Part 1 was directly hydrogenated to obtain oxymorphone (XI).

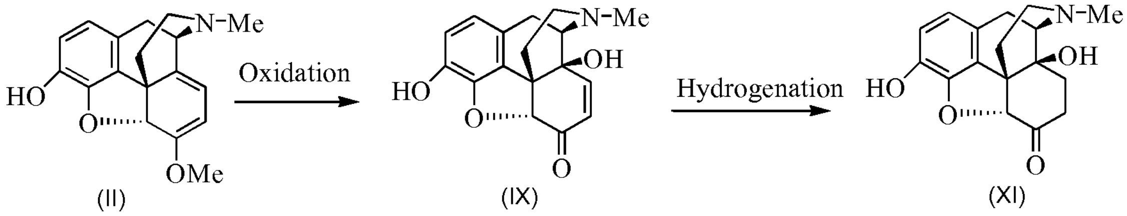


(II) (IX) (XI)

Alternative b)

Part 2 was cooled at 5 - 10 °C and then ammonia (200 mL) was added until pH 8.5, 14-hydroxymorphinone was precipitated; the suspension was stirred for 4 hours at 2 °C and the solid was filtered and washed with water (55 mL). After drying, 39 g of 14-hydroxymorphinone (IX) as light brown solid was obtained. HPLC assay: 78%. HPLC purity: 99%. The yield was 76 %.

Part 1 was diluted with 2-propanol (80 mL) and 1.25 g of Pd/C (50% wet) was added. The mixture was hydrogenated at 20 psi during 20 hours at 70 °C. 1.25 g of Pd/C (50% wet) was added again and the mixture was hydrogenated at 20 psi during 48 hours at 70 °C. The catalyst was filter over celite, washed with water (2 x 25 mL) and the filtrate was diluted with water (200 mL). 50% aqueous sodium hydroxide was added at 40 - 50 °C until pH 8 and this suspension was stirred for 30 minutes at 50 °C. The pH was adjusted at 9 with more 50% NaOH and the mixture was cooled at rt and stirred during 1 hour. The product was collected by filtration and washed with water (3x25 mL) and 2-propanol (25 mL). After drying, 35.62 g of oxymorphone was obtained. HPLC assay: 88.5% HPLC purity: 96.7%, 2.3% oxymorphol. Yield 78%.

Step 3. Acetylation of oxymorphone (XI) to 3,14-diacetyloxymorphone (I).

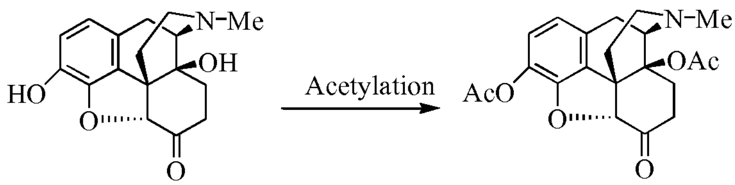


(XI) (I)

Oxymorphone (10 g, 88.5% assay. 0.029 mol) was suspended in toluene (50 mL) and acetic anhydride (13.5 g, 0.132 mol) was added. The suspension was stirred for about 2.5 hours at 70°C; the solid was dissolved after 5 minutes of reaction. The solution was cooled at rt and the solvent was evaporated; acetic acid and acetic anhydride was distilled azeotropically with fresh toluene. 14.4 g of orange solid was obtained after evaporation at dryness. HPLC assay: 67%. HPLC purity: 87.6%. The yield was 85.3%

The product was purified by crystallization in 2-methyltetrahydrofuran affording 7.74 g of pure product (HPLC

purity 97.2% and HPLC assay 91.6%; ABUK: 0.22%). Purification yield: 73.5%.

Comparative example 4

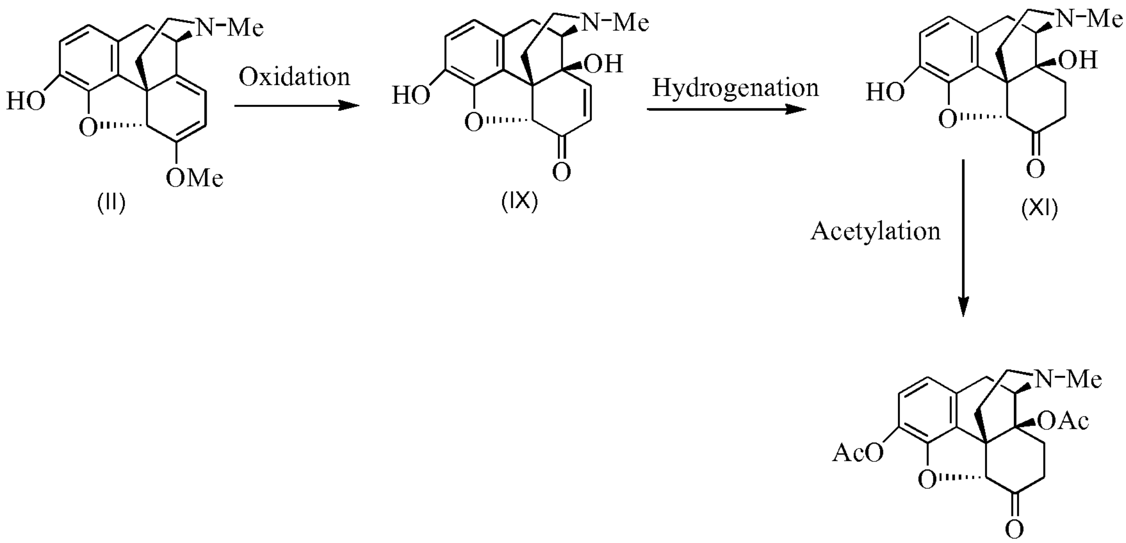


(II) (IX) (XI) (I)

Step 1.

Oripavine (40 g, 0.1345 mol) was added in 20 minutes to a solution of water (21.2 g) and formic acid (110.4 g) at 20 - 25 °C. The mixture was stirred at 20 - 25 °C until complete dissolution (15 - 20 minutes). 30% Hydrogen peroxide (16.8 g) was added dropwise in one hour at about 20 °C and the solution was stirred for 16 h at 15 - 20 °C. Then, it was cooled at 5 - 10 °C and then ammonia (200 mL) was added until pH 8.5, 14-hydroxymorphinone was precipitated; the suspension was stirred for 4 hours at 2 °C and the solid was filtered and washed with water (55 mL). After drying, 39 g of 14-hydroxymorphinone (IX) as light brown solid was obtained. HPLC assay: 78%. HPLC purity: 99%. The yield was 76 %.

Step 2. Hydrogenation of isolated 14-hydroxymorphinone (IX) to oxymorphone (XI) following the teaching of WO2008/072018 (previous art)

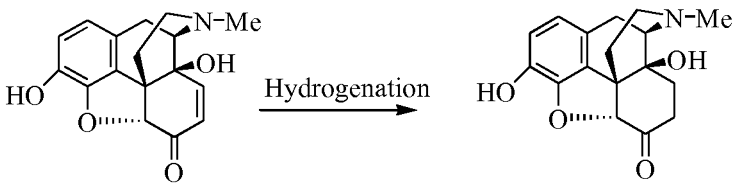


(IX) (XI)

14-hydroxymorphinone (IX) (38.8 g, 78% assay. 0.101 mol) was dissolved in water (100 mL) and acetic acid (29 g). 1.6 g of Pd/C (50% wet) was added. The mixture was hydrogenated at 40 psi during 4 hours at 30 °C. The catalyst was filter over celite, washed with water (30 mL) and the filtrate was cooled at 0 - 2 °C. 13% aqueous ammonium hydroxide was added at 2 °C until pH 9.5 and this suspension was stirred for 30 minutes at 2 °C. The product was collected by filtration and washed with water (3x25 mL). After drying, 30 g of oxymorphone was obtained. HPLC assay: 91.6% HPLC purity: 94.6%, 5% oxymorphol. Yield 90%

Step 3. Acetylation of oxymorphone (XI) to 3,14-diacetyloxymorphone (I).

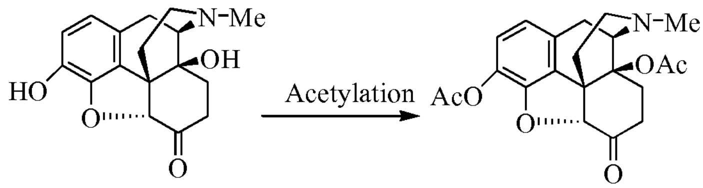


(XI) (I)

Oxymorphone (10 g, 91.6% assay. 0.030 mol) was suspended in toluene (50 mL) and acetic anhydride (13.5 g, 0.132 mol) was added. The suspension was stirred for about 2.5 hours at 70°C; the solid was dissolved after 5 minutes of reaction. The solution was cooled at rt and the solvent was evaporated; acetic acid and acetic anhydride was distilled azeotropically with fresh toluene. 13 g of orange solid was obtained after evaporation at dryness. HPLC assay: 84%. HPLC purity: 91.5%. The yield was 93.3%

The product was purified by crystallization in 2-methyltetrahydrofuran affording 9.51 g of pure product (HPLC purity 98.1% and HPLC assay 96%; ABUK: 0.23%). Purification yield: 83.5%

Comparative example 5

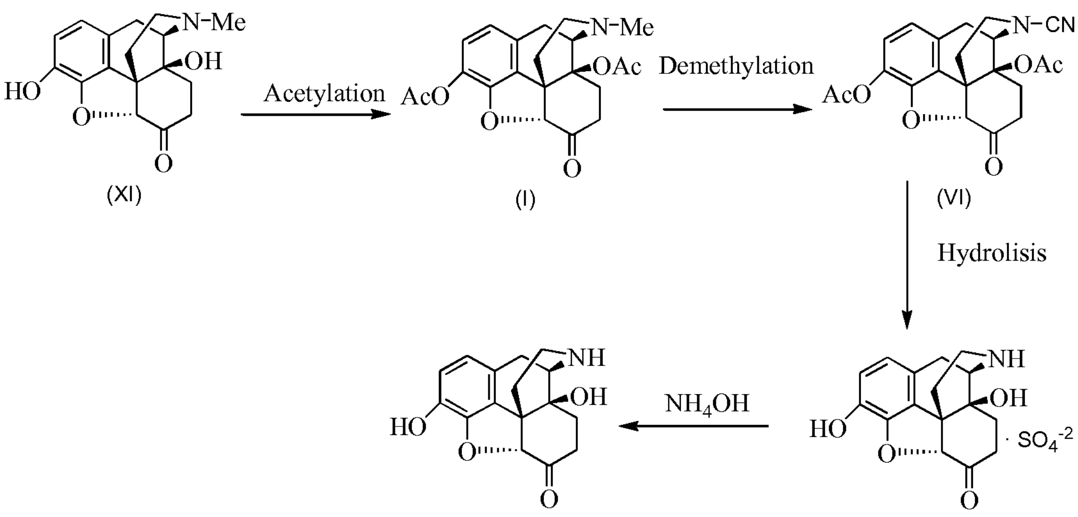


(XI) (I) (VI) (VIII) (VIIIa)

Step1. Acetylation of pure oxymorphone (XI) to 3,14-diacetyloxymorphone (I).

Oxymorphone (39.1 g, ≥98% assay. 0.127 mol) was suspended in toluene (196 mL) and acetic anhydride (43 g, 0.422 mol) was added. The suspension was stirred for about 3 hours at 70°C; the solid was dissolved after 60 minutes of reaction. The solution was cooled at rt and the solvent was evaporated; acetic acid and acetic anhydride was distilled azeotropically with fresh toluene. 50 g of beige solid was obtained after evaporation at dryness. HPLC assay: 99%. HPLC purity: 98%. The yield was 100%

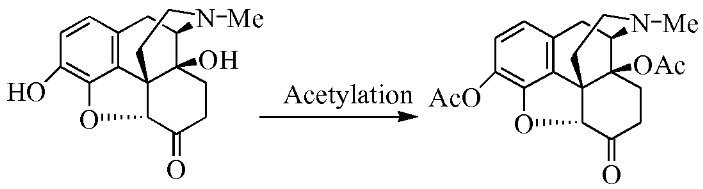


(XI) (I)

Step 2. Demethylation of 3,14-diacetyloxymorphone (I) (from comparative example 8) *N*-Cyano-3,14-diacetyloxymorphone (VI)

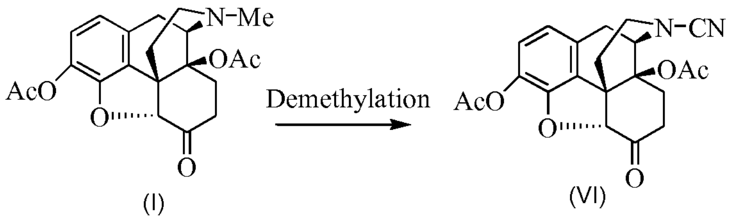


(I) (VI)

3,14-Diacetyloxymorphone (I) (49 g, ≥98% assay. 0.125 mol) was suspended in acetonitrile (270 mL) and cyanogen bromide (27.4 g, 0.258 mol) was added. The suspension was stirred for about 4 hours at 75°C; the solid was dissolved during the reaction. The solution was diluted with acetonitrile (54 mL) and cooled at 2 °C for 3 - 4 hours. A solid was precipitated which was filtered and washed with acetonitrile; 40 g of white solid was obtained after drying. HPLC assay: 97%. HPLC purity: 97%. The yield was 78.5 %.

Step 3. Hydrolysis of *N*-cyanoderivative (VI) (from comparative example 9) to noroxymorphone (VIII)

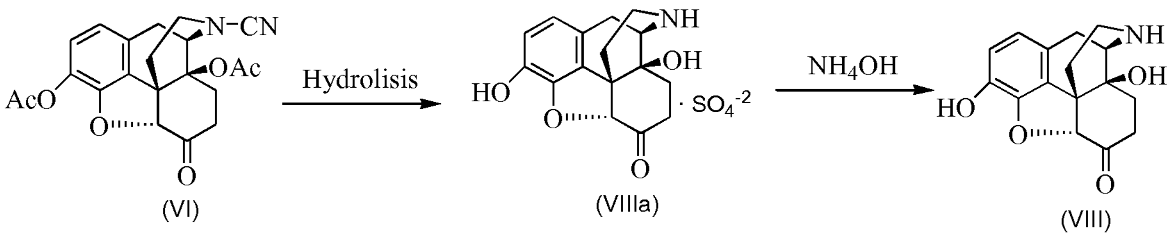


(VI) (VIIIa) (VIII)

*N*-Cyano-3,14-Diacetyloxymorphone (VI) (40 g, 97% assay. 0.98 mol) was suspended in 25% sulfuric acid (960 mL). The suspension was heated at reflux and stirred for about 18 hours at 100°C; the solid was dissolved after 2 hours of reaction. The reaction was cooled at 15 - 20 °C and then 25% ammonia (500 mL) was added until pH 9, precipitating noroxymorphone; the suspension was stirred for 1 hour at 5 °C and the solid was filtered and washed with water (2 x 75 mL) and ethanol (75 mL). After drying, 28 g of dihydrate noroxymorphone was obtained (as light brown solid). HPLC assay: 98%. HPLC purity: 98%. The yield was 86.7%

Comparative example 6

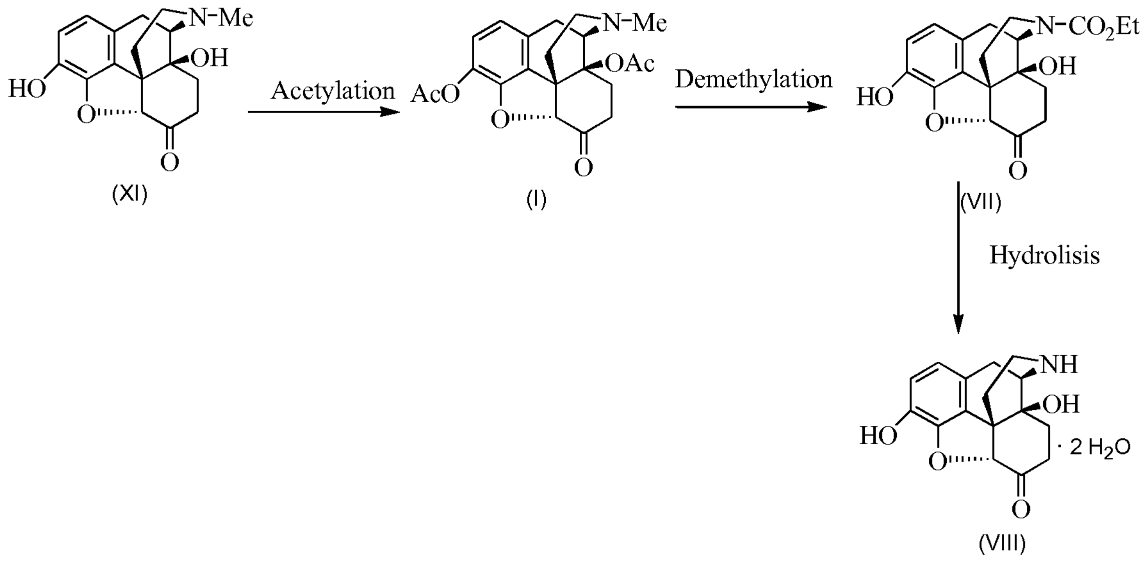


(XI) (I) (VII) (VIII)

Step1. Acetylation of pure oxymorphone (XI) to 3,14-diacetyloxymorphone (I).

Oxymorphone (39.1 g, ≥98% assay. 0.127 mol) was suspended in toluene (196 mL) and acetic anhydride (43 g, 0.422 mol) was added. The suspension was stirred for about 3 hours at 70°C; the solid was dissolved after 60 minutes of reaction. The solution was cooled at rt and the solvent was evaporated; acetic acid and acetic anhydride was distilled azeotropically with fresh toluene. 50 g of beige solid was obtained after evaporation at dryness. HPLC assay: 99%. HPLC purity: 98%. The yield was 100%

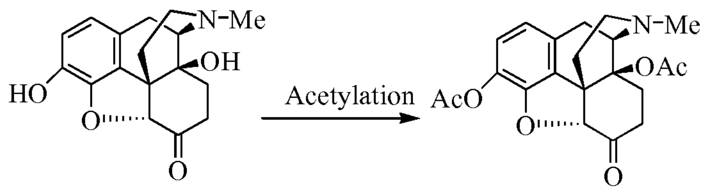


(XI) (I)

Step 2. Demethylation of 3,14-diacetyloxymorphone (I) (from comparative example 8) to a carbamate (VII)

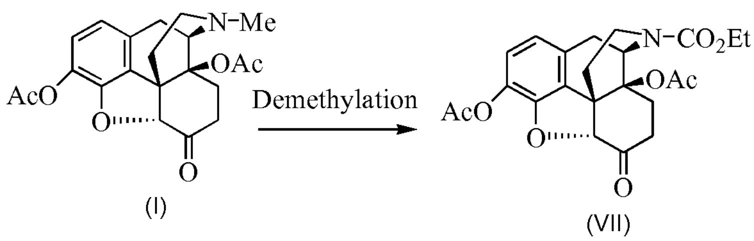


(I) (VII)

3,14-diacetyloxymorphone (I) (5.0 g, 0.0130 mol) was added in one portion to a suspension of sodium bicarbonate (1.1 g, 0.0130 mol) in dichloroethane (17 mL). The mixture was cooled with an ice bath and a solution of ethyl chloroformate (3.1 g, 0.0288 mol) in dichloroethane (8.0 mL) was dropwise added. Once the addition was done, the resulting cloudy solution was stirred at room temperature for 30 minutes and then refluxed until completion (48 hours). The reaction mixture was cooled to room temperature and washed with $NaHCO_3$ aqueous saturated solution (2 x 25 mL). The organic layer was dried, filtered and evaporated affording VII (5.5 g) as a yellow solid (96% yield).

Step 3. Hydrolysis of the carbamate (from Comparative example 10) to noroxymorphone (VIII)

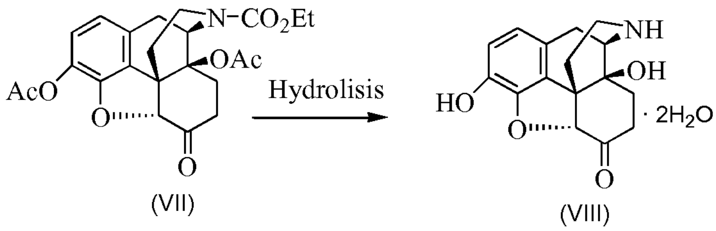


(VII) (VIII)

A suspension of carbamate (VII) (5.5 g, 0.0124 mol) in 40% sulphuric acid (60 mL) was heated at 95 -100 °C overnight. The resulting solution was cooled to 75 °C, charcoal was added and the black suspension was stirred for 30 minutes at this temperature. The mixture was filtered through celite and the cake rinsed with hot water (60 °C). The clarified solution was brought to pH 8.5 with 25% ammonia solution keeping the temperature between 25-30 °C. Once basification was done, the resulting solution was cooled to 10 °C and stirred at this temperature for 1 hour; the suspension was filtered, rinsed with water and ethanol and dried affording VIII as a brownish solid (3.1 g, 96% HPLC purity and 96% HPLC assay). Yield 78%.

**EXAMPLES**

Example 1. Acetylation of oripavine to 3-acetyloripavine (III). Preparation of 3-acetyl oripavine (III).

To a solution of oripavine (50 g, 0.1684 mol) in dry toluene (100 mL) was added acetic anhydride (86 g, 0.8418 mol) under a water bath. The resulting suspension was stirred at room temperature for 1 hour and then the solvent was evaporated. Several co-evaporations with toluene were performed and the residue was dissolved in a mixture of ethyl acetate/water and the pH was brought to 8.5 with a 33% $K_2CO_3$ aqueous solution. The suspension was filtered and the obtained solid was rinsed with water and dried affording (III) (51.52 g, 81% yield) as a brown solid with 98.5% purity and 90% in the HPLC assay.

Example 2. Acetylation of oripavine followed by oxidation (one-pot). Preparation of 3-acetyl-14-hydroxymorphinone (IV) from oripavine in one pot. *Isolation of 3-acetyl-14-hydroxymorphinone (IV) with 20% $Na_2CO_3$ solution (40 g scale).*

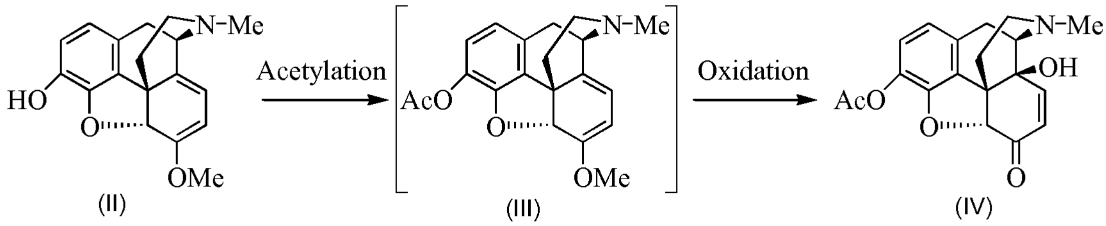


(II) (III) (IV)

Oripavine (40.0 g, 0.1345 mol) was added portion-wise to acetic anhydride (86.4 g, 0.8463 mol) under an ice bath in 40 minutes and the resulting suspension was stirred for 2 hours at room temperature. Water (16.0 g) was added (ice bath) in half an hour and the mixture was stirred at ambient temperature for 2 additional hours. Hydrogen peroxide (16.0 g, 0.1411 mol) was added dropwise at around 15 °C in 1.5 hours and the reaction was left stirring overnight at room temperature. The resulting dark solution was diluted with ethyl acetate and water and the pH was brought to 8.5 with 20% $Na_2CO_3$ aqueous solution. The suspension was filtered and the obtained solid was rinsed with water and dried affording (IV) (36.4 g, 75% yield) as a brownish solid with more than 98% purity and 95% HPLC assay.

Example 3. Acetylation of oripavine followed by oxidation (one-pot). Preparation of 3-acetyl-14-hydroxymorphinone (IV) from oripavine in one pot. *Isolation of 3-acetyl-14-hydroxymorphinone (IV) with 25% ammonia solution.*

Oripavine (40.0 g, 0.1345 mol) was added portion-wise to acetic anhydride (86.4 g, 0.8463 mol) under an ice

bath in 40 minutes and the resulting suspension was stirred for 2 hours at room temperature. Water (16.0 g) was added (ice bath) in half an hour and the mixture was stirred at ambient temperature for 2 additional hours. Hydrogen peroxide (16.0 g, 0.1411 mol) was added dropwise at around 10 °C in 1.5 hours and the reaction was left stirring overnight at room temperature. The resulting dark solution was diluted with ethyl acetate and water and the pH was brought to 8.5 with 25% aqueous ammonia solution. The suspension was filtered and the obtained solid was rinsed with water and dried affording (IV) (35.9 g) as a brownish solid with 95.5% purity and 94% HPLC assay. (Yield=73.5%)

Example 4. Acetylation of oripavine followed by oxidation (one-pot). Preparation of 3-acetyl-14-hydroxymorphinone (IV) from oripavine in one pot. *Isolation of 3-acetyl-14-hydroxymorphinone (IV) with 33% $K_2CO_3$ solution.*

Oripavine (80.0 g, 0.269 mol) was added portion-wise to acetic anhydride (173 g, 1.693 mol) at room temperature in 40 minutes and the resulting suspension was stirred for 2 hours the same temperature. Water (32.0 g) was added in 50 minutes and the mixture was stirred for 2 additional hours. Hydrogen peroxide (32.0 g, 0.282 mol) was added dropwise at around 15 °C in 1.2 hours and the reaction was left stirring overnight at room temperature. The resulting dark solution was diluted with ethyl acetate and water and the pH was brought to 8.5 with 33% $K_2CO_3$ aqueous solution. The suspension was filtered and the obtained solid was rinsed with water and dried affording (IV) (71.8 g, 76% yield) as a brownish solid with more than 98% purity and 96% HPLC assay. Example 5. Acetylation of oripavine followed by oxidation (one-pot). Preparation of 3-acetyl-14-hydroxymorphinone (IV) from oripavine in one pot. *Isolation of 3-acetyl-14-hydroxymorphinone (IV) with 50% $K_2CO_3$ solution.*

Oripavine (80.0 g, 0.269 mol) was added portion-wise to acetic anhydride (173 g, 1.693 mol) at room temperature in 20 minutes and the resulting suspension was stirred for 2 hours the same temperature. Water (32.0 g) was added in 37 minutes and the mixture was stirred for 2 additional hours. Hydrogen peroxide (32.0 g, 0.282 mol) was added dropwise at around 15 °C in 74 minutes and the reaction was left stirring overnight at room temperature. The resulting dark solution was diluted with ethyl acetate and water and the pH was brought to 8.5 with 50% $K_2CO_3$ aqueous solution. The suspension was filtered and the obtained solid was rinsed with water and dried affording (IV) (70.7 g, 73% yield) as a brownish solid with 99% purity and 95% HPLC assay.

Example 6. Acetylation of oripavine followed by oxidation (one-pot). Preparation of 3-acetyl-14-hydroxymorphinone (IV) from oripavine in one pot. *Isolation of 3-acetyl-14-hydroxymorphinone (IV) with 33% $K_2CO_3$ solution (120 g scale).*

Oripavine (120.0 g, 0.404 mol) was added portion-wise to acetic anhydride (260 g, 2.543 mol) at room temperature in 21 minutes and the resulting suspension was stirred for 2 hours the same temperature. Water (48 g) was added in 55 minutes and the mixture was stirred for 2 additional hours. Hydrogen peroxide (48 g, 0.424 mol) was added dropwise at around 15 °C in 74 minutes and the reaction was left stirring overnight at room temperature. The resulting dark solution was diluted with ethyl acetate and water and the pH was brought to 8.5 with 33% $K_2CO_3$ aqueous solution. The suspension was filtered and the obtained solid was rinsed with water and dried affording (IV) (113.3 g, 78% yield) as a brownish solid with 98% purity and 95% HPLC assay.

Example 7. Acetylation of oripavine followed by oxidation and hydrogenation (one-pot). Preparation of 3-acetyloxymorphone (V) from oripavine in one pot.

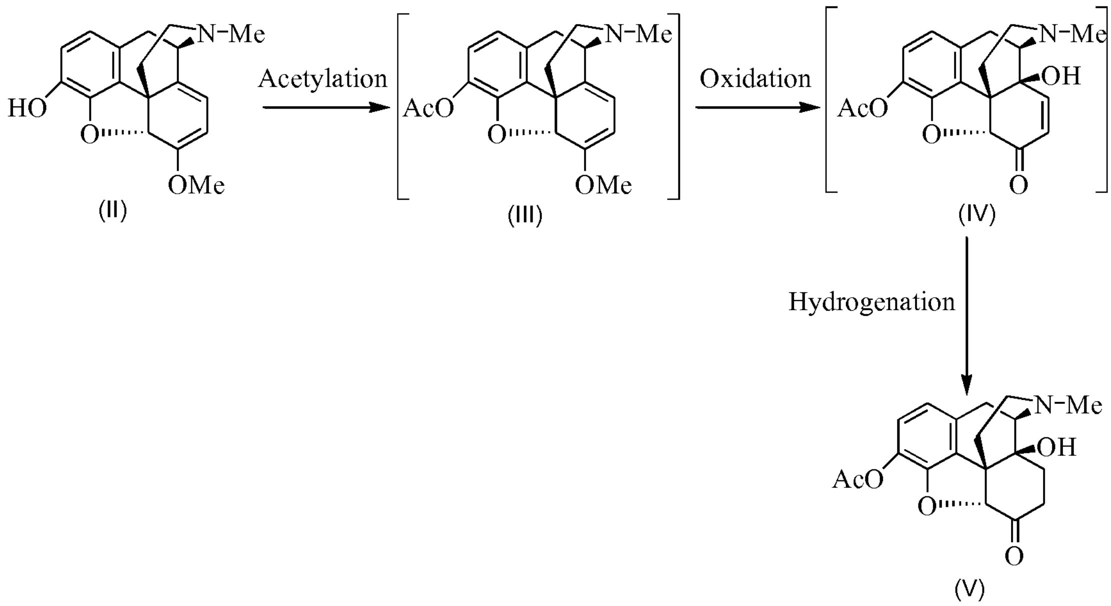


(II) (III) (IV) (V)

Oripavine (30.0 g, 0.1010 mol) was added portion-wise to acetic anhydride (65 g, 0.6336 mol) at room temperature in 15 minutes and the resulting suspension was stirred for 2 hours the same temperature. Water (12.0 g) was added in 22 minutes and the mixture was stirred for 2 additional hours. Hydrogen peroxide (12.0 g, 0.1061 mol) was added dropwise at around 15 °C in 44 minutes and the reaction was left stirring overnight at room temperature. The resulting dark solution was diluted with water (20 mL), Pd/C (5%) was added and the mixture was hydrogenated under pressure for several hours. Filtration through celite was carried out followed by rinsing the cake with a 1:1 AcOH:$H_2O$ mixture. The resulting solution was evaporated until slurry was obtained, then it was diluted with ethyl acetate and water and the pH was brought to 8.5 with 33% $K_2CO_3$ aqueous solution. The emulsion was filtered through celite and layers were separated. The celite cake was rinsed with ethyl acetate and used to back extract the aqueous layer. The combined organic extracts were dried, filtered and evaporated until a brown foam was obtained (35.5 g, around 27 g of (V) were detected with 82% purity and 78% yield).

Example 8. Acetylation of oripavine followed by oxidation, hydrogenation and acetylation (one pot). Preparation of 3,14-diacetyloxymorphone (I) from oripavine in one pot.

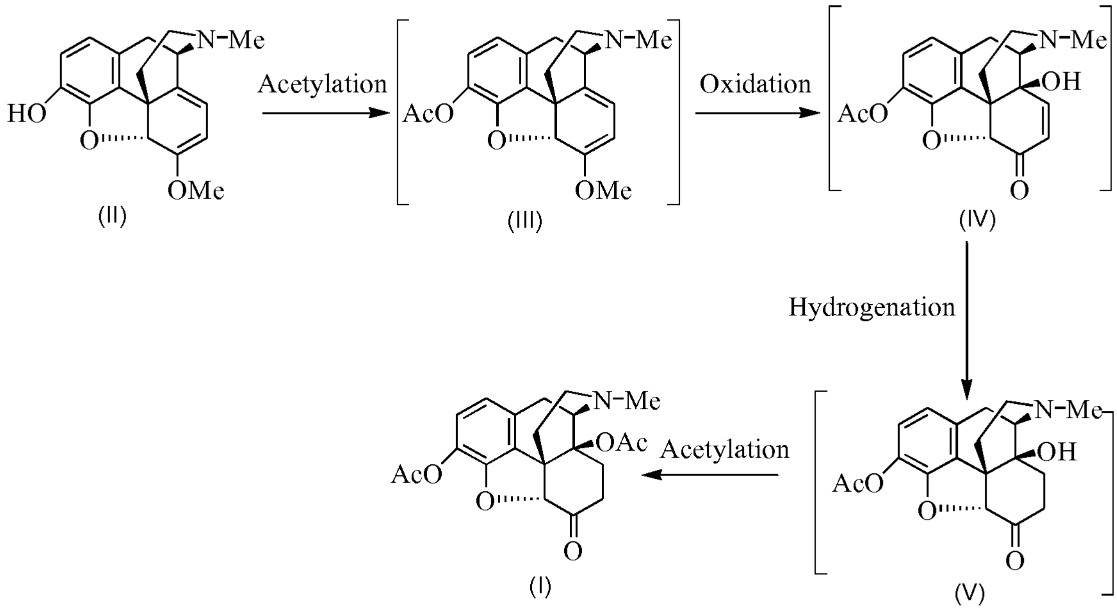


(II) (III) (IV) (I) (V)

Oripavine (30.0 g, 0.1010 mol) was added portion-wise to acetic anhydride (65 g, 0.6336 mol) at room temperature in 15 minutes and the resulting suspension was stirred for 2 hours the same temperature. Water (12.0 g) was added in 26 minutes and the mixture was stirred for 2 additional hours. Hydrogen peroxide (12.0 g, 0.1061 mol) was added dropwise at around 15 °C in 35 minutes and the reaction was left stirring overnight at room temperature. The resulting dark solution was diluted with water (20 mL), Pd/C (5%) was added and the mixture was hydrogenated under pressure for several hours. Filtration through celite was carried out followed by rinsing of the cake with a 1:1 AcOH:$H_2O$ mixture and the solvent was azeotropically evaporated through several co-evaporations with toluene affording a dark toluene solution that contained around 25% of product. To the previous solution, acetic anhydride (22 g, 0.216 mol) was added and the mixture was heated at 75 °C until completion. The solvent was partially evaporated under reduced pressure and the residue was co-evaporated with dry toluene until 53.5 g of slurry was achieved. 232 g of (2-MeTHF) and charcoal were added. The black suspension was heated for 30 minutes, filtered, the obtained mixture was refluxed again until total dissolution of the product and then distilled off around 90% of solvent at atmospheric pressure. The resulting slurry was cooled to -5 °C in 5 hours and the mixture was left between -5°C to 0°C for 15 hours. The suspension was filtered and rinsed with diisopropyl ether affording 21.5 g of a brownish solid (52% yield) with 95% purity and 94% in the HPLC assay.

Example 9. Oxidation of 3-acetyloripavine (III) to 3-acetyl-14-hydroxymorphinone (IV).

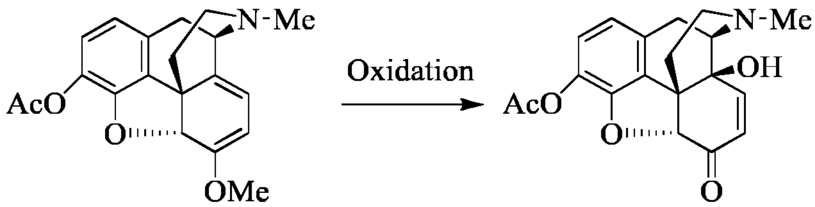


(III) (IV)

A suspension of (III) (5.0 g, 0.0148 mol) in acetic anhydride (7.5 g, 0.0737 mol) was prepared. Water (1.8 g) was then added under an ice bath and the mixture was stirred at room temperature for 2 hours. Hydrogen peroxide (1.8 g, 0.0155 mol) was dropwise added at around 15 °C and the reaction was left stirring overnight. The mixture was diluted with ethyl acetate and water, cooled with an ice bath and the pH was brought to 8.5 with 33% $K_2CO_3$ aqueous solution. The suspension was filtered and the obtained solid was rinsed with water and dried affording a brownish solid (4.34 g, 67% yield) with 94% purity and 75% in the HPLC assay.

Example 10. Hydrogenation of 3-acetyl-14-hydroxymorphinone (IV) to 3-acetyloxymorphone (V).

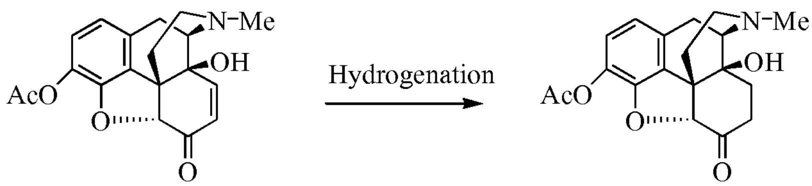


(IV) (V)

A solution of (IV) (71.8, 0.210 mol), Pd/C (2.0 g, 2.8%) in a mixture of acetic acid (263 g)/water (7 g) was hydrogenated under pressure for several hours. Filtration through celite was carried out followed by rinsing with a 1:1 AcOH:$H_2O$ mixture. The resulting solution was evaporated until slurry was obtained, then it was diluted with ethyl acetate (750 mL) and water (165 mL) and the pH was brought to 8.5 with 33% $K_2CO_3$ aqueous solution. The formed emulsion was filtered through celite and layers were separated. The celite cake was rinsed with ethyl acetate (200 mL) and used to back extract the aqueous layer. The combined organic extracts were dried, filtered and evaporated until a brown foam was obtained (70.6 g) with more than 97% purity of (V), 98% HPLC assay, 0.9% 3-acetyl-14-hydroxymorphinone and 0.5% oxymorphone. Yield = 95%.

Example 11. Hydrogenation of 3-acetyloxymorphinone (IV) to 3-acetyloxymorphone (V).

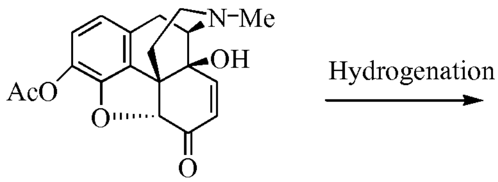


(IV) (V)

A solution of (IV) (70.7, 0.207 mol), Pd/C (1.9 g, 2.5%) in a mixture of acetic acid (280 g)/water (7.5 g) was hydrogenated under pressure for several hours. Filtration through celite was carried out followed by rinsing with a 1:1 AcOH:$H_2O$ mixture. The resulting solution was evaporated until slurry was obtained, then it was diluted with toluene (400 mL) and water (150 mL) and the pH was brought to 8.5 with 33% $K_2CO_3$ aqueous solution. The emulsion was filtered through celite and layers were separated. The celite cake was rinsed with toluene (150 mL) and used to back extract the aqueous layer. The combined organic extracts were dried, filtered and evaporated until a solution of (V) in toluene was obtained (68.8 g of product in 260 g of toluene). HPLC analysis of the solution revealed 96% purity, 0.4% oxymorphinone and 0.4% oxymorphone with a 97% yield.

Example 12. Hydrogenation of 3-acetyloxymorphinone (IV) to 3-acetyloxymorphone (V).

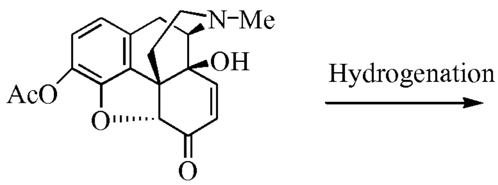


(IV) (V)

A solution of (IV) (100.4 g, 0.294 mol), Pd/C (2.6 g, 2.5%) in a mixture of acetic acid (360 g)/water (40 g) was hydrogenated under pressure for several hours. Filtration through celite was carried out followed by rinsing with a 1:1 AcOH:$H_2O$ mixture. The resulting solution was evaporated until slurry was obtained, then it was diluted with ethyl acetate (700 mL) and water (200 mL) and the pH was brought to 8.5 with 33% $K_2CO_3$ aqueous solution. The emulsion was filtered through celite and layers were separated. The celite cake was rinsed with ethyl acetate (200 mL) and used to back extract the aqueous layer. The combined organic extracts were dried, filtered, toluene was added (100 g) and the solvent was evaporated. The resulting slurry was co-evaporated with toluene in order to remove the remaining ethyl acetate affording a solution of (V) in toluene (100 g of product in 385 g of toluene). HPLC analysis of the solution revealed 93% purity, 1.2% oxymorphinone and 1.2% oxymorphone. Yield = 99%.

Example 13. Hydrogenation of 3-acetyloxymorphinone (IV) followed by acetylation to 3,14-diacetyloxymorphone (I) (one-pot).

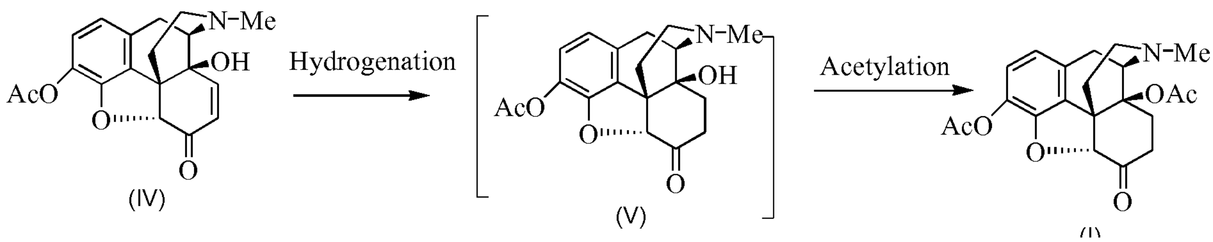


(IV) (V) (I)

A solution of (IV) (46 g, 0.1349 mol), Pd/C (0.75 g, 2.5%) in a mixture of 180 g of acetic acid and 20 g of water

was hydrogenated under pressure (maximum of 3 bars) for several hours. Filtration through celite was carried out followed by 35 g of a 1:1 mixture of acetic acid and water. Solvent was azeotropically evaporated by sequential co-evaporations with toluene until 60 g (45 g of V was quantified by HPLC) of slurry was obtained with 94% purity and 1.2% oxymorphinone and 1.5% oxymorphone detected.

To the previous residue (46 g of V, 0.1312 mol), dry toluene (190 g) was added together with acetic anhydride (26.8 g, 0.2624 mol) and the mixture was heated between 70 to 75 °C until completion. The solvent was partially evaporated under reduced pressure and the residue was co-evaporated with dry toluene until 75 g of a dark brown solid was achieved. Recrystallization in 2-MeTHF gave a slurry that was cooled to -5 °C in 5 hours and the mixture was left between -5°C to 0°C for 15 hours. The dark brown suspension was filtered and rinsed with diisopropyl ether (70 g) affording 44.6 g of a dark brown solid that after HPLC anaylsis revealed 97% purity, 89% in the assay and ABUK: 0.3%. Yield of (I) was 76%.

Example 14. Acetylation of 3-acetyloxymorphone (V) to 3,14-diacetyloxymorphone (I).

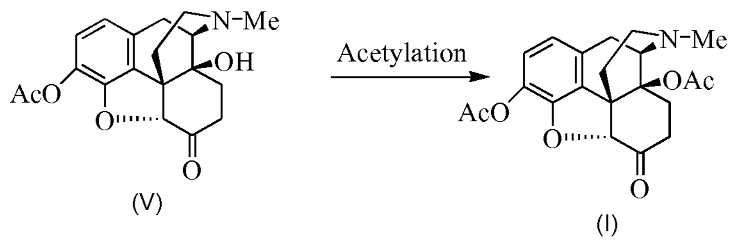


(V) (I)

A solution of (V) (70.6 g, 0.206 mol and isolated using ethyl acetate), acetic anhydride (38 g, 0.371 mol) in dry toluene (350 mL) was heated to 75 °C until completion. The solvent was partially evaporated under reduced pressure and the residue was co-evaporated with dry toluene until 100 g of slurry was achieved. Recrystallization in 2-MeTHF gave a slurry that was cooled to -5 °C in 5 hours and the mixture was left between -5°C to 0°C for 15 hours. The suspension was filtered and rinsed with diisopropyl ether affording (I) (70 g, 87% yield) as a light yellow solid with 98% purity and 98% HPLC assay. ABUK: 0.29%.

Example 15. Acetylation of 3-acetyloxymorphone (V) to 3,14-diacetyloxymorphone (I).

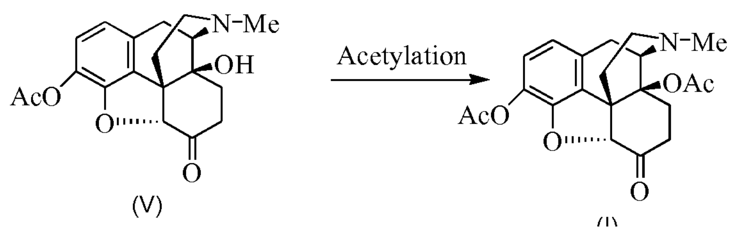


(V) (I)

A solution of (V) (14.3 g, 0.04169 mol (coming from (III) in one pot and isolated using ethyl acetate), acetic anhydride (7.7 g, 0.075 mol) in dry toluene (75 mL) were heated to 75 °C until completion. The solvent was partially evaporated under reduced pressure and the residue was co-evaporated with dry toluene until 29 g of slurry was achieved. Recrystallization in 2-MeTHF gave a slurry that was cooled to -5 °C in 5 hours and the mixture was left between -5°C to 0°C for 15 hours. The suspension was filtered and rinsed with diisopropyl ether affording 17 g of a yellow solid with 94% purity and 92% HPLC assay. Yield of (I) was 97%. ABUK: 1.58%

Example 16. Acetylation of 3-acetyloxymorphone (V) to 3,14-diacetyloxymorphone (I).

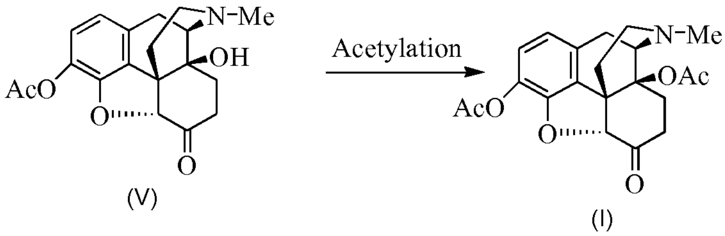


(V) (I)

A solution of (V) (27 g, 0.0787 mol (coming from oripavine in one pot and isolated using ethyl acetate), acetic anhydride (14.5 g, 0.142 mol) in dry toluene (90 mL) were heated to 75 °C until completion. The solvent was partially evaporated under reduced pressure and the residue was co-evaporated with dry toluene until 51 g of slurry was achieved. Recrystallization in 2-MeTHF gave a slurry that was cooled to -5 °C in 5 hours and the mixture was left between -5°C to 0°C for 15 hours. The suspension was filtered and rinsed with diisopropyl ether affording 26 g of a yellow solid with 95% purity and 94% HPLC assay. Yield of (I) was 80%. ABUK: 0.90%.

Example 17. Acetylation of 3-acetyloxymorphone (V) to 3,14-diacetyloxymorphone (I).

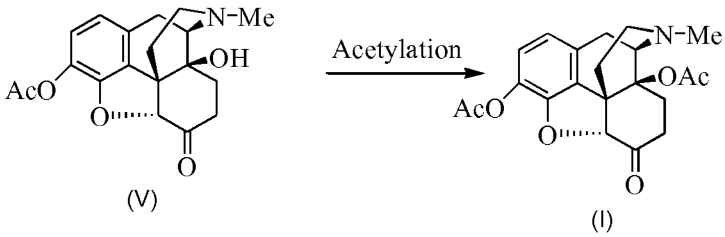


(V) (I)

A solution of (V) (100 g in 300 g of toluene) and acetic anhydride (53.6 g, 0.525 mol) were heated to 75 °C until completion. The solvent was partially evaporated under reduced pressure and the residue was co-evaporated with dry toluene until 147 g of slurry was achieved. Recrystallization in 2-MeTHF gave a slurry that was cooled to -5 °C in 5 hours and the mixture was left between -5°C to 0°C for 15 hours. The suspension was filtered and rinsed with diisopropyl ether affording 101.8 g of a yellow solid with 98% purity and 98% HPLC assay. Yield of (I) was 89%. ABUK: 0.29%.

Example 18. Acetylation of 3-acetyloxymorphone (V) to 3,14-diacetyloxymorphone (I).

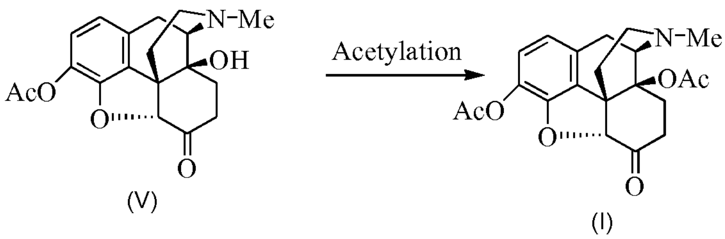


(V) (I)

A solution of (V) (100 g in 385 g of toluene coming from a solvent swap between ethyl acetate and toluene done in the work up of the previous reaction) and acetic anhydride (53.8 g, 0.527 mol) were heated to 75 °C until completion. The solvent was partially evaporated under reduced pressure and the residue was co-evaporated with dry toluene until 154 g of slurry was achieved. Recrystallization in 2-MeTHF gave a slurry that was cooled to -5 °C in 5 hours and the mixture was left between -5°C to 0°C for 15 hours. The suspension was filtered and rinsed with diisopropyl ether affording 96.7 gof a yellow solid with 98% purity and 98% HPLC assay. Yield of (I) was 84%. ABUK: 0.29%

Example 19. Oxidation of 3-acetyloripavine (III) followed by hydrogenation (one-pot) to yield 3-acetyloxymorphone (V).

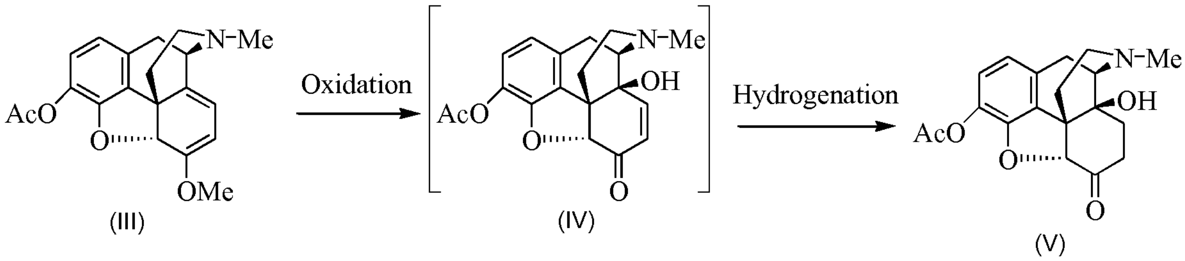


(III) (IV) (V)

A suspension of (III) (25.0 g, 0.0738 mol) in acetic anhydride (38 g, 0.03687 mol) was prepared. Water (8.8 g) was then added under an ice bath and the mixture was stirred at room temperature for 2 hours. Hydrogen peroxide (8.8 g, 0.0774 mol) was dropwise added at around 15 °C in 50 minutes and the reaction was left stirring overnight. The resulting dark solution was diluted with water (15 mL), Pd/C (5%) was added and the mixture was hydrogenated under pressure for several hours. Filtration through celite was carried out followed by rinsing with a 1:1 AcOH:$H_2O$ mixture. The resulting solution was evaporated until slurry was obtained, then it was diluted with ethyl acetate and water and the pH was brought to 8.5 with 33% $K_2CO_3$ aqueous solution. The resulting emulsion was filtered through celite and layers were separated. The celite cake was rinsed with ethyl acetate and used to back extract the aqueous layer. The combined organic extracts were dried, filtered and evaporated until a brown foam was obtained (24 g, around 14 g of (V) were detected with 70% purity). 10% of oxymorphone and 3.6% of oxymorphinone were also observed. Yield of (I) was 57%

Example 20. Oxidation of 3-acetyloripavine followed by hydrogenation and acetylation (one-pot) to yield 3,14-diacetyloxymorphone (I).

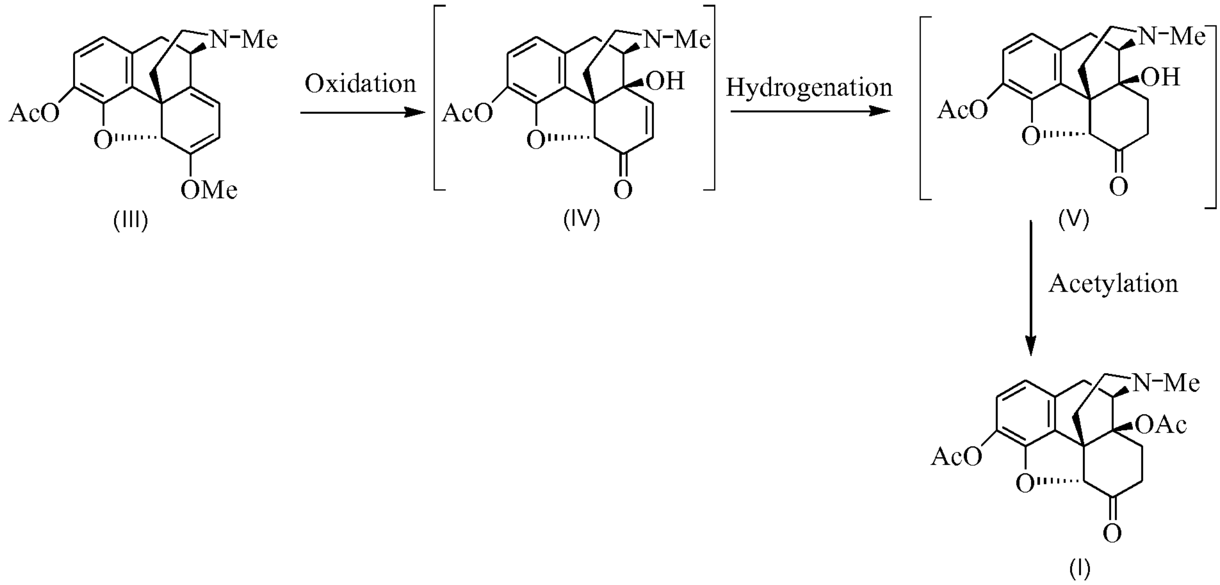


(III) (IV) (V) (I)

A suspension of (III) (25.0 g, 0.0738 mol) in acetic anhydride (38 g, 0.03687 mol) was prepared. Water (8.8 g) was added under an ice bath and the mixture was stirred at room temperature for 2 hours. Hydrogen peroxide (8.8 g, 0.0774 mol) was dropwise added at around 15 °C and the reaction was left stirring overnight. The resulting dark solution was diluted with water (15 mL), Pd/C (5%) was added and the mixture was hydrogenated under pressure for several hours. Filtration through celite was carried out followed by rinsing with a 1:1 AcOH:$H_2O$ mixture. The solvent was azeotropically evaporated through several co-evaporations with toluene affording a dark toluene solution that contained around 25% of product. To the previous solution, acetic anhydride (19 g, 0.186 mol) was added and the mixture was heated at 75 °C until completion. The solvent was partially evaporated under reduced pressure and the residue was co-evaporated with dry toluene until 50 g of slurry was achieved. Recrystallization in 2-MeTHF gave a slurry that was cooled to -5 °C in 5 hours and the mixture was left between -5°C to 0°C for 15 hours. The suspension was filtered and rinsed with diisopropyl ether affording 20.2 g of a brownish solid with 94% purity and 78% HPLC assay. Yield of (I) was 55% ABUK: 1.36%.

Example 21. Demethylation of 3,14-diacetyloxymorphone (I) to *N*-Cyano-3,14-diacetyloxymorphone (VI)

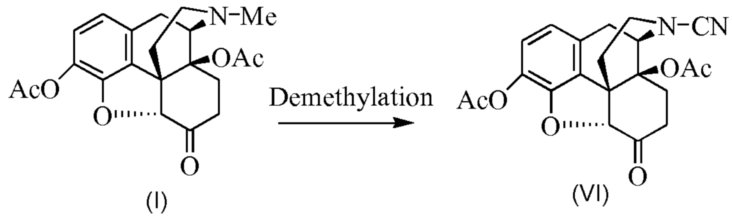


(I) (VI)

3,14-Diacetyloxymorphone (I) (96 g, ≥98% assay. 0.244 mol) was suspended in acetonitrile (576 mL) and cyanogen bromide (38.3 g, 0.361 mol) was added. The suspension was stirred for about 3 hours at 70 °C. The reaction was cooled at 2 °C for 3 - 4 hours. A solid was precipitated which was filtered and washed with acetonitrile; 86.5 g of white solid was obtained after drying. HPLC assay: 95.5%. HPLC purity: 97%, less than 0.1% of *N*-Cyano-3,14-diacetyloxymorphinone was quantified. The yield was 85.3 %.

Example 22. Hydrolysis of *N*-cyanoderivative (VI) to noroxymorphone (VIII)

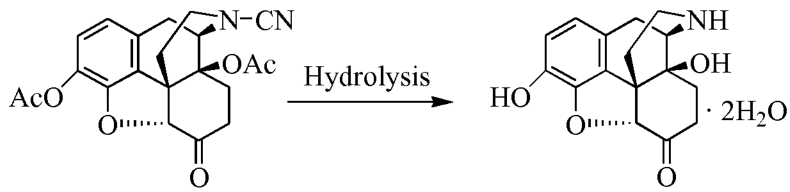


(VI) (VIII)

*N*-Cyano-3,14-Diacetyloxymorphone (VI) (89.3 g, 91.8% assay. 0.207 mol) was suspended in 25% sulfuric acid (1068 mL). The suspension was heated at reflux and stirred for about 18 hours at 100°C; the solid was dissolved after 2 hours of reaction. The reaction was cooled at 65 °C, charcoal (5%) was added and the suspension was stirred at about 60 °C during 1 hour. Charcoal was filtered over celite and washed with water (230 mL). 25% ammonia (about 650 mL) was added to the filtrate until pH 9 at about 50 °C and noroxymorphone was precipitated. The suspension was cooled and stirred for 1 hour at 5 °C and the solid was filtered and washed with water (2 x 160 mL) and ethanol (160 mL). After drying (50 °C in vacuo), 61.76 g of monohydrated noroxymorphone was obtained (as light brown solid). HPLC assay: 99%. HPLC purity: 99.7%. ABUK 770 ppm. The yield was 96.7%

Example 23. Hydrolysis of *N*-cyanoderivative (VI) to noroxymorphone (VIII) (with sulphate salt isolation)

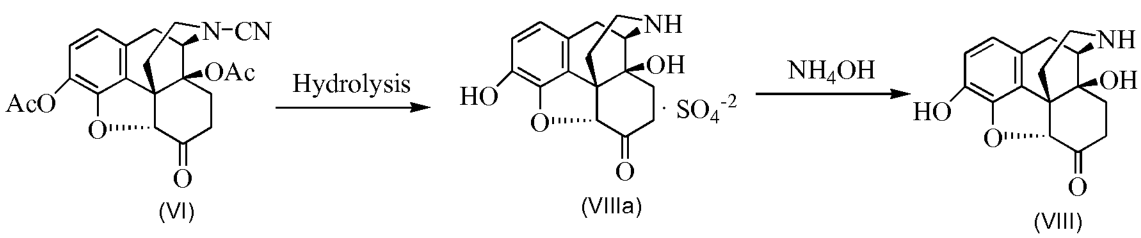


(VI) (VIIIa) (VIII)

*N*-Cyano-3,14-Diacetyloxymorphone (VI) (84.2 g, 96% assay. 0.204 mol) was suspended in 25% sulfuric acid (1045 mL). The suspension was heated at reflux and stirred for about 18 hours at 100°C; the solid was dissolved after 2 hours of reaction. The reaction was cooled at 65 °C, charcoal (5%) was added and the suspension was stirred at about 60 °C during 1 hour. Charcoal was filtered over celite and washed with water (75 mL). The filtrate was cooled at 0 - 5 °C and stirred at this temperature for about 16 hours, the sulphate salt of noroxymorphone was precipitated. The white

solid was filtered and washed with 25% sulfuric acid (25 mL). The wet salt (78 g containing 53.6 g of noroxymorphone base) was dissolved with water (535 mL) at 60 - 65 °C and charcoal (5%) was added. The suspension was stirred at about 60 °C during 1 hour. Charcoal was filtered over celite and washed with water (15 mL) and the filtrate was neutralized with 25% ammonia (about 22 mL) until pH 9 at about 50 °C, noroxymorphone was precipitated. The suspension was cooled and stirred for 1 hour at 10 °C; the solid was filtered and washed with water (2 x 60 mL) and ethanol (60 mL). After drying (50 °C), 57.2 g of dihydrated noroxymorphone was obtained (as white solid). HPLC assay: 99%. HPLC purity: 99.8%. ABUK 120 ppm. The yield was 86%

Example 24. Purification of noroxymorphone (VIII) through its sulphate salt

10 g of noroxymorphone (7.3% water, grey solid: 99.66% purity, ABUK 450 ppm) was dissolved in 25% $H_2SO_4$ (160 mL) at about 80 °C. The solution was cooled at 0 - 5 °C in about 3 hours and the suspension was stirred at this temperature during 5 hours. The sulphate precipitated at 50 - 55 °C as a white crystalline solid. The solid was filtered and washed with cool solvent (5 mL).
12.7 g of wet solid was obtained

The obtained wet solid was dissolved in water (90 mL) at about 60 °C and the solution was treated with charcoal (0.4 g) for about 30 minutes. The charcoal was filtered and washed with hot water (15 mL). The filtrate was alkalinized with ammonia until pH 8 at 50 °C and noroxymorphone was precipitated. The suspension was cooled at 10 °C and stirred at this temperature during 1 hour. The solid was filtered and washed with water (2 x 20 mL) and ethanol (20 mL).

After drying, 9.2 g of dihydrated noroxymorphone was obtained (as white solid; water 11.6%). HPLC Purity: 99.88%. ABUK: 307 ppm. Yield: 88%

Example 25. Optional purification of noroxymorphone (VIII) through its hydrochloride 10 g of noroxymorphone (7.3% water, grey solid: 99.66% purity, ABUK 450 ppm) was dissolved in 16% HCl (160 mL) at about 90 °C. The solution was cooled at 0 - 5 °C in about 3 hours and the suspension was stirred at this temperature during 5 hours. The hydrochloride precipitated at 60 - 65 °C as a white crystalline solid. The solid was filtered and washed with cool solvent (5 mL).

12.6 g of wet solid was obtained.

The obtained wet solid was dissolved in water (90 mL) at about 60 °C and the solution was treated with charcoal (0.4 g) for about 30 minutes. The charcoal was filtered and washed with hot water (15 mL). The filtrate was alkalinized with ammonia until pH 8 at 50 °C and noroxymorphone was precipitated. The suspension was cooled at 10 °C and stirred at this temperature during 1 hour. The solid was filtered and washed with water (2 x 20 mL) and ethanol (20 mL).

After drying, 9.57 g of dihydrated noroxymorphone was obtained (as white solid; water 10.7%).
HPLC Purity: 99.93%. ABUK: 167 ppm. Yield: 92%

Example 26. Reaction of 3,14-diacetyloxymorphone to yield (VII).

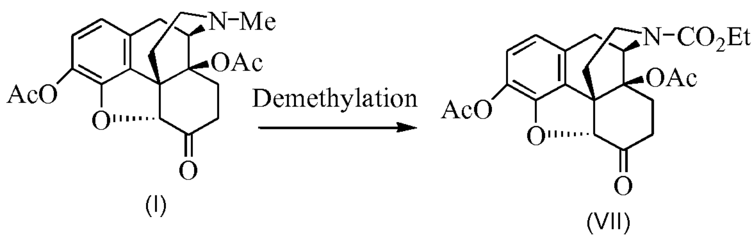


(I) (VII)

3,14-diacetyloxymorphone (I) (5.0 g, 0.0129 mol) was added in one portion to a suspension of sodium bicarbonate (1.2 g, 0.0143 mol) in dichloroethane (16 mL). The mixture was cooled with an ice bath and a solution of ethyl chloroformate (3.2 g, 0.0299 mol) in dichloroethane (8.0 mL) was dropwise added. Once the addition was done, the resulting cloudy solution was stirred at room temperature for 30 minutes and then refluxed until completion (96 hours). The reaction mixture was cooled to room temperature and washed with $NaHCO_3$ aqueous saturated solution (2 x 25 mL). The organic layer was dried, filtered and evaporated affording (VII) (5.78 g, 94% yield) as a yellow solid.

Example 27. Reaction of 3,14-diacetyloxymorphone to yield a carbamate. (VII).

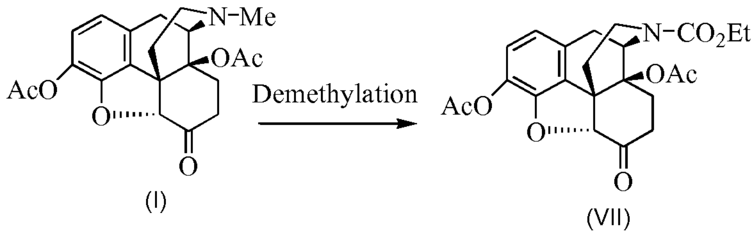


(I) (VII)

3,14-diacetyloxymorphone (I) (5.0 g, 0.0129 mol) was added in one portion to a suspension of sodium bicarbonate (1.2 g, 0.0143 mol) in dichloroethane (16 mL). The mixture was cooled with an ice bath and a solution of ethyl chloroformate (3.2 g, 0.0299 mol) in dichloroethane (8.0 mL) was dropwise added. Once the addition was done, the resulting cloudy solution was stirred at room temperature for 30 minutes and then refluxed until completion (48 hours). The reaction mixture was cooled to room temperature and washed with $NaHCO_3$ aqueous saturated solution (2 x 25 mL). The organic layer was dried, filtered and evaporated affording (VII) (6.85 g) as a yellow gum that was directly submitted to the next hydrolysis step.

Example 28. Hydrolysis of the carbamate (VII) to yield noroxymorphone (VIII)

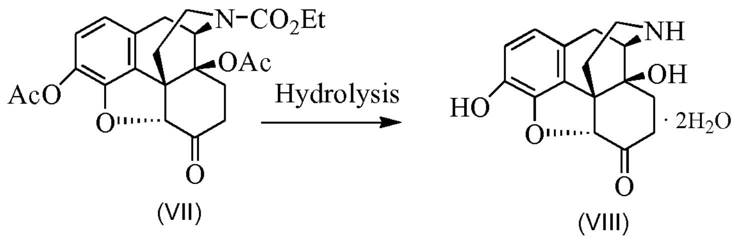


(VII) (VIII)

A suspension of *N*-ethyl carbamate- diacetyloxymorphone (VII) (5.4 g, 0.0122 mol) in 40% sulphuric acid (60 mL) was heated at 95 °C overnight. The resulting solution was cooled to 75 °C, charcoal was added and the black suspension was stirred for 30 minutes at the mentioned temperature. The mixture was filtered through celite and the cake rinsed with hot water (60 °C). The clarified solution was brought to pH 8.5 with 25% ammonia solution keeping the temperature between 60-70 °C. Once basification was done, the resulting solution was cooled to 0 °C in 3 hours; the suspension was filtered, rinsed with water and ethanol and dried affording 2.97 g of a brownish solid with 96% purity, 91.5% HPLC assay and 78% yield.

Example 29. Allylation of noroxymorphone (VIII) to naloxone

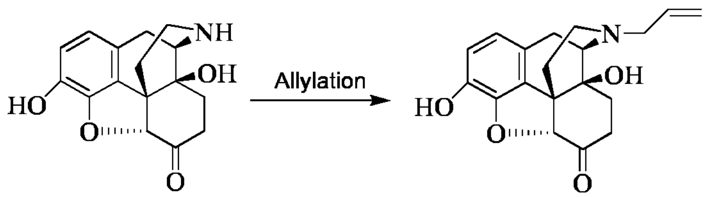


(VIII) (Naloxone)

Noroxymorphone (17.5 g, 10.74% of water. 0.054 mol) was suspended in *N*-methylpyrrolidone (61.5 g) at rt. Potassium hydrogen carbonate (9.03 g, 0.090 mol) was added and the suspension was stirred at 20 - 25 °C for 10 minutes. Allyl bromide (10.15 g, 0.084 mol) was added dropwise and the reaction was stirred at 30 °C during 2 hours. After that, water (350 mL) was added and naloxone was precipitated and the pH was adjusted at 9 with 33% aqueous potassium carbonate. The suspension was stirred at 0 - 5 °C for 3 hours and the solid was filtered and washed with water (35 mL). After drying, 17.43 g of naloxone was obtained (as white solid).HPLC assay: 93%. HPLC Purity: 98%. Yield: 91%

Example 30. Alkylation of noroxymorphone (VIII) to naltrexone

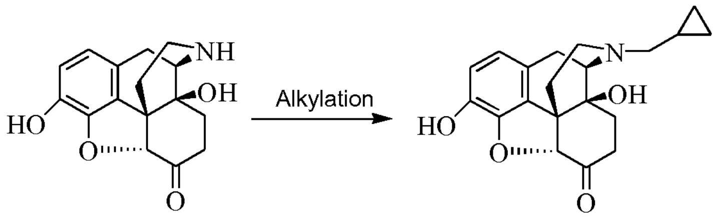


(VIII) (Naltrexone)

Noroxymorphone (7.18 g, 7% of water. 0.023 mol) was suspended in *N*-methylpyrrolidone (26.5 g) at rt. Potassium hydrogen carbonate (3.88 g, 0.039 mol) was added and the suspension was stirred at 20 - 25 °C for 10 minutes. Cyclopropylmethyl-bromide (4.06 g, 0.030 mol) was added dropwise and then, the reaction was stirred at 50 °C during 5 hours. After that, 10% aqueous sodium chloride (150 mL) was added and naltrexone was precipitated and the pH was adjusted at 9 with 33% aqueous potassium carbonate. The suspension was stirred at 0 - 5 °C for 2 hours and the solid was filtered and washed with water (15 mL).

After drying, 6.9 g of naltrexone was obtained (as white solid).
HPLC assay: 98.8%. HPLC Purity: 98.5%. Yield: 86%

Example 31. Acylation of noroxymorphone to N,O-bis(cyclobutanecarbonyl)noroxymorphone

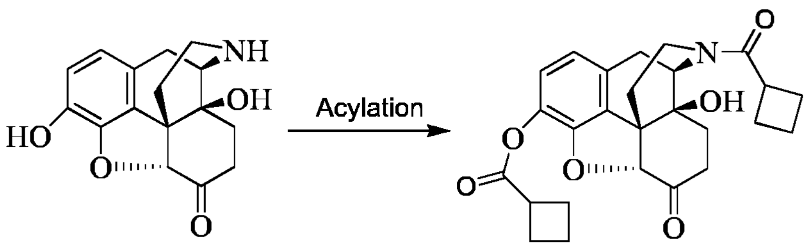


(VIII) (N,O-bis(cyclobutanecarbonyl)noroxymorphone)

Noroxymorphone (10 g, 10% of water. 0.031 mol) was suspended in THF (90 mL) at rt. The suspension was cooled at 15 °C and triethylamine (9.8 g, 0.97 mol) was added dropwise and the reaction was stirred for about 5 minutes. Cyclobutanecarbonyl chloride (9.9 g, 0.835 mol) was added at 15 °C in 30 minutes and the suspension was stirred during 1 hour. Salts were filtered and washed with THF and the filtrate was evaporated giving an oil which was dissolved in dichloromethane (100 mL) and washed with 5% aqueous sodium bicarbonate. The organic layer was dried over sulphate and the solvent was evaporated affording a sticky solid that was triturated with isopropyl ether (100 mL). The solid was filtered and washed with ether.

After drying (50°C in vacuo), 12.5 g of N,$O^3$-bis(cyclobutanecarbonyl)noroxymorphone was obtained (as white fine solid). HPLC purity: ≥98. Yield: 87%

Example 32. Reduction of N,O-bis(cyclobutanecarbonyl)noroxymorphone to nalbuphine

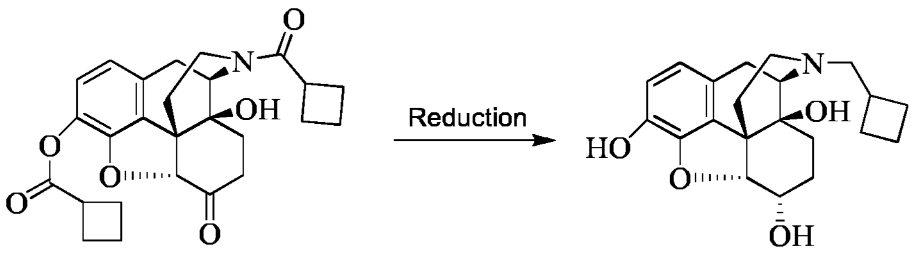


(N,O-bis(cyclobutanecarbonyl)noroxymorphone) (Nalbuphine)

A solution of 1 g (2.22 mmol) of N,O[3]-bis(cyclobutanecarbonyl)noroxymorphone in 10 mL of THF was added over a solution of borane:THF complex (50 mL) at 5 °C in 20 minutes. The reaction was heated at reflux for about 8 hours and 3M hydrochloric acid (20 mL) was added and then, this mixture was refluxed for 1 hour. The suspension was cooled at rt and salts were filtered. The filtered was diluted with dichloromethane and neutralized with 25% aqueous ammonia until pH 8. The organic layer was dried over sulphate and the solvent was evaporated affording 0.6 g of nalbuphine (as solid white). Yield 75%.The product was crystallized in methanol giving 0.15 g of pure product.

## Claims

1. A process for preparing 3,14-diacetyloxymorphone (I) comprising the steps of:

    a) reacting oripavine (II) with an acetylating agent to yield compound (III),

    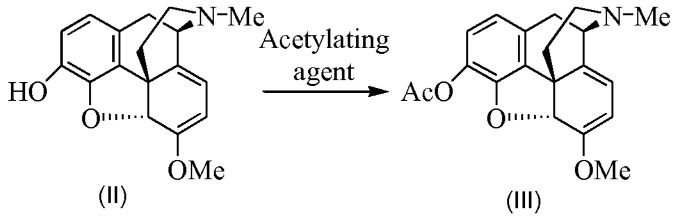
    

    (II) (III)

    b) reacting compound (III) with an oxidizing agent to yield compound (IV),

    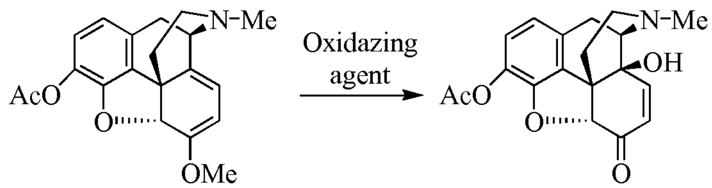
    

    (III) (IV)

    c) reacting compound (IV) with a hydrogenating agent to yield compound (V),

    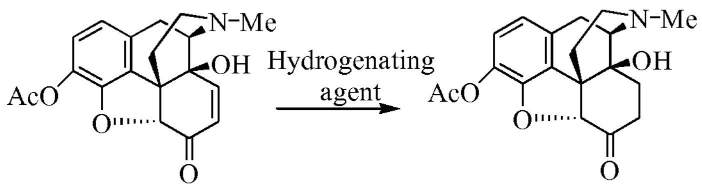
    

    (IV) (V)

    d) reacting compound (V) with an acetylating agent to yield 3,14-diacetyloxymorphone (I).

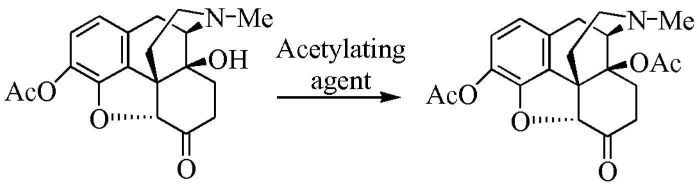


(V) (I)

2. A process according to claim 1 wherein step b) is carried out after step a) without isolating or purifying product (III).

3. A process according to any one of claims 1 to 2 wherein step d) is carried out after step c) without isolating or purifying product (V).

4. A process according to any one of claims 1 to 3 wherein product (IV) is isolated from the reaction mixture resulting from step b) before carrying out step c).

5. A process according to any one claims 1 to 4 wherein step a) is carried out using acetic anhydride as acetylating agent.

6. A process according to any one claims 1 to 5 wherein step b) is carried out using a mixture of hydrogen peroxide and acetic acid as oxidizing agent.

7. A process according to any one claims 1 to 6 wherein step c) is carried out using a hydrogen gas in the presence of a Pd/C catalyst as hydrogenating agent.

8. A process according to any one claims 1 to 7 wherein step d) is carried out using acetic anhydride as acetylating agent.

9. A process for preparing noroxymorphone (preferably as a hydrate) (VIII) wherein 3,14-diacetyloxymorphone (I) is prepared according to the process of claims 1 to 8 and 3,14-diacetyloxymorphone (I) then subjected to a process comprising the steps of:

e) reacting compound (I) with a demethylating agent to yield either compound (VI) or compound (VII), and
f) reacting either compound (VI) or compound (VII) with a hydrolising agent to yield noroxymorphone (VIII).

10. A process according to claim 9 wherein step e) is carried out using cyanogen bromide as demethylating to yield compound (VI).

11. A process according to claim 9 wherein step e) is carried out using a chloroformate as demethylating to yield compound (VII).

12. A process according to claim 11 wherein the chloroformate is ethyl chloroformate.

13. A process according to any one of claims 9 to 12 wherein step f) is carried out using an acid having a pKa lower than 5.

14. A process according to claim 13 wherein the acid is sulfuric acid.

15. A process for the preparation of naloxone, naltrexone, nalbuphine, nalfurafine or nalmefene from oripavine (II) comprising the preparation of 3,14-diacetyloxymorphone (I) according to the process of claims 1 to 8, the subsequent conversion of (I) to noroxymorphone (VIII) as described in claims 9 to 14 and the subsequent conversion of noroxymorphone (VIII) to naloxone, naltrexone, nalbuphine, nalfurafine or nalmefene.

**Patentansprüche**

1. Verfahren zum Herstellen von 3,14-Diacetyloxymorphon (I), welches die Schritte umfasst:

a) Umsetzen von Oripavin (II) mit einem acetylierenden Mittel, um Verbindung (III) hervorzubringen,

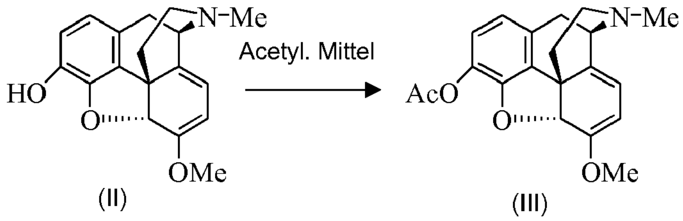


(II) (III)

b) Umsetzen von Verbindung (III) mit einem oxidierenden Mittel, um Verbindung (IV) hervorzubringen,

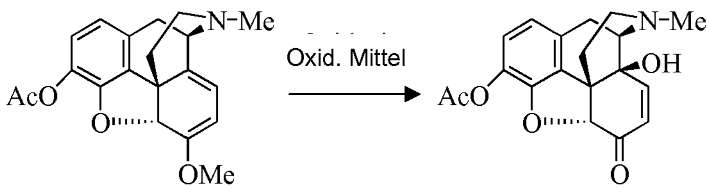


(III) (IV)

c) Umsetzen von Verbindung (IV) mit einem hydrierenden Mittel, um Verbindung (V) hervorzubringen,

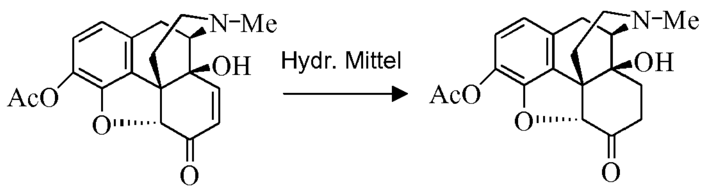


(IV) (V)

d) Umsetzen von Verbindung (V) mit einem acetylierenden Mittel, um 3,14-Diacetyloxymorphon (I) hervorzubringen.

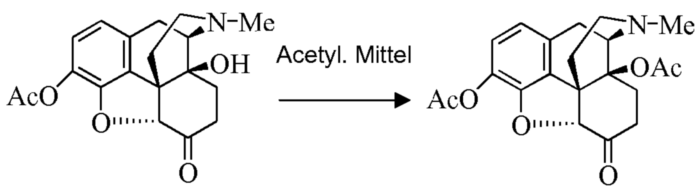


(V) (I)

2. Verfahren nach Anspruch 1, wobei Schritt b) nach Schritt a) ohne Trennen oder Reinigen von Produkt (III) durchgeführt wird.

3. Verfahren nach einem der Ansprüche 1 bis 2, wobei Schritt d) nach Schritt c) ohne Trennen oder Reinigen von Produkt (V) durchgeführt wird.

4. Verfahren nach einem der Ansprüche 1 bis 3, wobei Produkt (IV) vor dem Durchführen von Schritt c) von der sich in Schritt b) ergebenden Reaktionsmischung getrennt wird.

5. Verfahren nach einem der Ansprüche 1 bis 4, wobei Schritt a) unter Verwendung von Essigsäureanhydrid als acetylierendem Mittel durchgeführt wird.

6. Verfahren nach einem der Ansprüche 1 bis 5, wobei Schritt b) unter Verwendung einer Mischung aus Wasserstoffperoxid und Essigsäure als oxidierendem Mittel durchgeführt wird.

7. Verfahren nach einem der Ansprüche 1 bis 6, wobei Schritt c) unter Verwendung eines Wasserstoffgases in Gegenwart eines Pd/C-Katalysators als hydrierendem Mittel durchgeführt wird.

8. Verfahren nach einem der Ansprüche 1 bis 7, wobei Schritt d) unter Verwendung von Essigsäureanhydrid als acetylierendem Mittel durchgeführt wird.

9. Verfahren zum Herstellen von Noroxymorphon (vorzugsweise als Hydrat) (VIII), wobei 3,14-Diacetyloxymorphon (I) gemäß dem Verfahren nach Ansprüchen 1 bis 8 hergestellt wird, und 3,14-Diacetyloxymorphon (I) dann einem Verfahren unterzogen wird, welches die Schritte umfasst:

e) Umsetzen von Verbindung (I) mit einem demethylierenden Mittel, um entweder Verbindung (VI) oder Verbindung (VII) hervorzubringen, und
f) Umsetzen von entweder Verbindung (VI) oder Verbindung (VII) mit einem hydrolysierenden Mittel, um Noroxymorphon (VIII) hervorzubringen.

10. Verfahren nach Anspruch 9, wobei Schritt e) unter Verwendung von Bromcyan als demethylierendem Mittel durchgeführt wird, um Verbindung (VI) hervorzubringen.

11. Verfahren nach Anspruch 9, wobei Schritt e) unter Verwendung eines Chlorameisensäureesters als demethylierendem Mittel durchgeführt wird, um Verbindung (VII) hervorzubringen.

12. Verfahren nach Anspruch 11, wobei der Chlorameisensäureester Chlorameisensäureethylester ist.

13. Verfahren nach einem der Ansprüche 9 bis 12, wobei Schritt f) unter Verwendung einer Säure mit einem pKa-Wert von weniger als 5 durchgeführt wird.

14. Verfahren nach Anspruch 13, wobei die Säure Schwefelsäure ist.

15. Verfahren zur Herstellung von Naloxon, Naltrexon, Nalbuphin, Nalfurafin oder Nalmefen aus Oripavin (II), umfassend die Herstellung von 3,14-Diacetyloxymorphon (I) gemäß dem Verfahren nach Ansprüchen 1 bis 8, die anschließende Überführung von (I) in Noroxymorphon (VIII) wie in Ansprüchen 9 bis 14 beschrieben, und die anschließende Überführung von Noroxymorphon (VIII) in Naloxon, Naltrexon, Nalbuphin, Nalfurafin oder Nalmefen.

## Revendications

1. Procédé de préparation de 3,14-diacétyloxymorphone (I) comprenant les étapes suivantes :

a) faire réagir de l'oripavine (II) avec un agent d'acétylation pour fournir le composé (III),

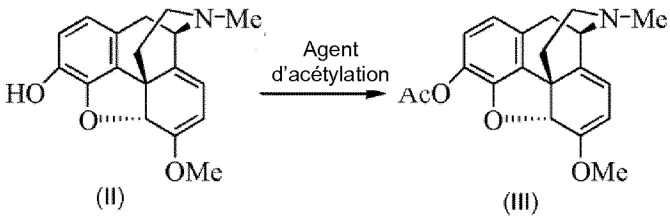


(II) (III)

b) faire réagir le composé (III) avec un agent d'oxydation pour fournir le composé (IV),

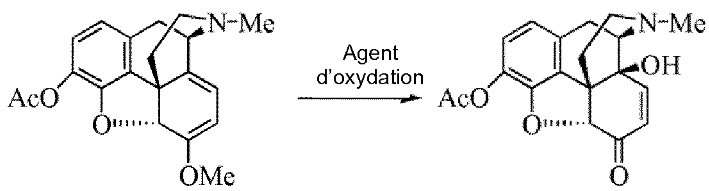


(III) (IV)

c) faire réagir le composé (IV) avec un agent d'hydrogénation pour fournir le composé (V),

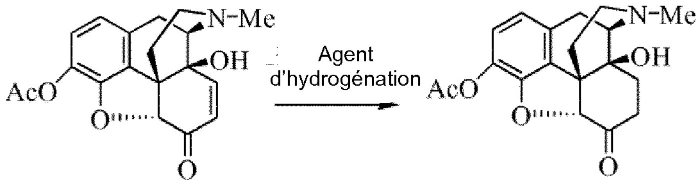


(IV) (V)

d) faire réagir le composé (V) avec un agent d'acétylation pour fournir la diacétyloxymorphone (I).

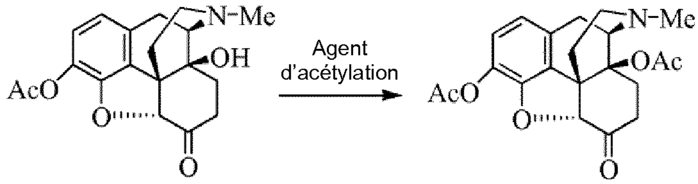


(V) (I)

2. Procédé selon la revendication 1 dans lequel l'étape b) est effectuée après l'étape a) sans isoler ou purifier le produit (III).

3. Procédé selon l'une quelconque des revendications 1 et 2 dans lequel l'étape d) est effectuée après l'étape c) sans isoler ou purifier le produit (V).

4. Procédé selon l'une quelconque des revendications 1 à 3 dans lequel le produit (IV) est isolé à partir du mélange réactionnel résultant de l'étape b) avant d'effectuer l'étape c).

5. Procédé selon l'une quelconque des revendications 1 à 4 dans lequel l'étape a) est effectuée à l'aide d'anhydride acétique comme agent d'acétylation.

6. Procédé selon l'une quelconque des revendications 1 à 5 dans lequel l'étape b) est effectuée à l'aide d'un mélange de peroxyde d'hydrogène et d'acide acétique comme agent d'oxydation.

7. Procédé selon l'une quelconque des revendications 1 à 6 dans lequel l'étape c) est effectuée à l'aide d'un gaz d'hydrogène en présence d'un catalyseur de Pd/C comme agent d'hydrogénation.

8. Procédé selon l'une quelconque des revendications 1 à 7 dans lequel l'étape d) est effectuée à l'aide d'anhydride acétique comme agent d'acétylation.

9. Procédé de préparation de noroxymorphone (de préférence sous forme d'hydrate) (VIII) dans lequel de la 3,14-diacétyloxymorphone (I) est préparée selon le procédé des revendications 1 à 8 et la 3,14-diacétyloxymorphone (I) est ensuite soumise à un procédé comprenant les étapes suivantes :

e) faire réagir le composé (I) avec un agent de déméthylation pour fournir soit le composé (VI) soit le composé (VII), et
f) faire réagir soit le composé (VI) soit le composé (VII) avec un agent d'hydrolyse pour fournir la noroxymorphone (VIII).

10. Procédé selon la revendication 9 dans lequel l'étape e) est effectuée à l'aide de bromure de cyanogène comme agent de déméthylation pour fournir le composé (VI).

11. Procédé selon la revendication 9 dans lequel l'étape e) est effectuée à l'aide d'un chloroformiate comme agent de déméthylation pour fournir le composé (VII).

12. Procédé selon la revendication 11 dans lequel le chloroformiate est le chloroformiate d'éthyle.

13. Procédé selon l'une quelconque des revendications 9 à 12 dans lequel l'étape f) est effectuée à l'aide d'un acide

ayant un pKa inférieur à 5.

**14.** Procédé selon la revendication 13 dans lequel l'acide est l'acide sulfurique.

**15.** Procédé de préparation de naloxone, de naltrexone, de nalbuphine, de nalfurafine ou de nalméfène à partir d'oripavine (II) comprenant la préparation de 3,14-diacétyloxymorphone (I) selon le procédé des revendications 1 à 8, la conversion subséquente de (I) en noroxymorphone (VIII) comme décrit dans les revendications 9 à 14 et la conversion subséquente de noroxymorphone (VIII) en naloxone, naltrexone, nalbuphine, nalfurafine ou nalméfène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 3254088 A
- US 4141897 A
- GB 2000137 B
- WO 2006084412 A
- WO 2009078989 A
- WO 2009122436 A
- EP 2121698 B1
- EP 2137190 B1
- EP 2377866 B1
- WO 9902529 A
- US 2806033 A
- ES 465073
- US 5071985 A
- WO 8000841 A
- WO 2007137782 A
- WO 2009111162 A
- US 2772270 A
- WO 2008072018 A, Johnson Matthey
- WO 2008048711 A1
- EP 2073636 B1
- WO 2008130553 A
- WO 2013188418 A
- WO 2011171712 A
- WO 2008118654 A
- WO 2005028483 A
- EP 2125824 B1
- WO 2014013313 A
- WO 2015015147 A

### Non-patent literature cited in the description

- **NORTON et al.** *J. Med. Chem.,* 1973, vol. 16, 556
- **OLOFSON et al.** *Tetrahedron Lett.,* 1977, 1567-1570
- **KRANIG et al.** *Arch. Pharm. Pharm. Med. Chem.,* 1996, vol. 329, 325-326
- **PENICK ; SCHWARTZ et al.** *J. Med. Chem.,* 1981, vol. 24, 1525
- **RICE et al.** *J. Org. Chem.,* 1998, vol. 63, 4392
- **ANDRE et al.** *Synth. Commun.,* 1992, vol. 22, 2313-2327
- **RICE et al.** *J. Med. Chem.,* 1978, vol. 21, 398
- **NIELSEN et al.** *Planta Med.,* 1983, vol. 48, 205-206
- **MILLGATE et al.** *Nature,* 2004, vol. 431, 413
- **COOP et al.** *J. Org. Chem.,* 1996, vol. 61, 6774
- *J.ORG.CHEM.,* 1998, vol. 63, 4392-4396
- **WEISS et al.** had previously disclosed its reduction to oxymorphone. *J. Org. Chem.,* 1957, vol. 22, 1505-1508
- **KOK et al.** *Org. Biomol. Chem.,* 2011, vol. 9, 1008-1011
- **KOK et al.** *RSC Advances,* 2012, vol. 2, 11318-11325
- **WEISS.** *J. Org. Chem.,* 1957, vol. 22, 1505-1508